# EUROPEAN PATENT SPECIFICATION

(11) **EP 0 429 570 B1**
(45) Date of publication and mention of the grant of the patent: **14.01.1998**
(21) Application number: 90905830.7
(22) Date of filing: 27.03.1990
(51) Int. Cl.: C12P 21/00, A61K 38/27, C07K 14/00

(54) **OSTEOINDUCTIVE COMPOSITIONS**
OSTEOINDUKTIVE ZUSAMMENSETZUNGEN
COMPOSITIONS OSTEOINDUCTRICES

(30) Priority: 28.03.1989 US 329610; 04.05.1989 US 347559; 23.06.1989 US 370544; 23.06.1989 US 370547; 23.06.1989 US 370549; 15.11.1989 US 437409; 17.11.1989 US 438919; 07.03.1990 US 490033
(43) Date of publication of application: 05.06.1991
(73) Proprietor: GENETICS INSTITUTE, INC., Cambridge, Massachusetts 02140 (US)
(72) Inventor: WANG, Elizabeth, A., Carlisle, MA 01741 (US); WOZNEY, John, M., Hudson, MA 01749 (US); ROSEN, Vicki, A., Brookline, MA 02146 (US); CELESTE, Anthony, J., Hudson, MA 01479 (US)
(74) Representative: VOSSIUS & PARTNER
(86) International application number: US9001630
(87) International publication number: WO9011366

(56) References cited:
- EP-A- 0 212 474
- WO-A-88/00205
- WO-A-89/09787
- WO-A-89/10409
- WO-A-91/05802
- US-A- 4 789 732
- Proc. Natl. Acad. Sci., vol. 85, no. 24, 1988; Wang, Elisabeth A et al.: "Purification and characterization of other distinct bone-inducing factors", see pages 9484, 9488

## Description

The present invention relates to proteins having utility in the formation of bone and/or cartilage. In particular the invention relates to a number of families of purified proteins, termed BMP-5, BMP-6 and BMP-7 protein families (wherein BMP is Bone Morphogenic Protein) and processes for obtaining them. These proteins exhibit the ability to induce cartilage and/or bone formation. They may be used to induce bone and/or cartilage formation and in wound healing and tissue repair.

WO 89/09787 discloses a partial nucleic acid and a partial amino acid sequence for a protein which is designated as "OP-1". Two different forms of the OP-1 sequence are disclosed, that is the genomic DNA with introns (Fig. 1A) with no indication of an open reading frame or the positions of the introns or exons and an undefined sequence region between positions 1880 and 1920, and in Figure 1B, a sequence that should correspond to part of the cDNA sequence with an overall length of 314 nucleotides. The partial amino acid sequence of OP-1 is disclosed on page 9.

WO 91/05802 discloses the genomic sequence of OP-1 from position 1 to 1822 and the derived amino acid sequence encoding the full length protein with a length of 431 amino acids. Furthermore, the region corresponding to the mature form of the protein is indicated in Figure 2 and also on page 26 and designated as OP1-18.

The invention provides a family of BMP-5 proteins. Purified human BMP-5 proteins are substantially free from other proteins with which they are co-produced, and characterized by an amino acid sequence comprising from amino acid #323 to amino acid #454 set forth in Table III. This amino acid sequence #323 to #454 is encoded by the DNA sequence comprising nucleotide #1665 to nucleotide #2060 of Table III. BMP-5 proteins may be further characterized by an apparent molecular weight of 28,000-30,000 daltons as determined by sodium dodecyl sulfate polyacrylamide gel electrophoresis (SDS-PAGE). Under reducing conditions in SDS-PAGE the protein electrophoreses with a molecular weight of approximately 14,000 - 20,000 daltons. It is contemplated that these proteins are capable of stimulating, promoting, or otherwise inducing cartilage and/or bone formation.

Human BMP-5 proteins of the invention may be produced by culturing a cell transformed with a DNA sequence containing the nucleotide sequence the same or substantially the same as the nucleotide sequence shown in Table III comprising nucleotide #699 to nucleotide #2060. BMP-5 proteins comprising the amino acid sequence the same or substantially the same as shown in Table III from amino acid # 323 to amino acid # 454 are recovered, isolated and purified from the culture medium.

The invention provides a family of BMP-6 proteins. Purified human BMP-6 proteins, substantially free from other proteins with which they are co-produced and are characterized by an amino acid sequence comprising acid #382 to amino acid #513 set forth in Table IV. The amino acid sequence from amino acid #382 to #513 is encoded by the DNA sequence of Table IV from nucleotide #1303 to nucleotide #1698. These proteins may be further characterized by an apparent molecular weight of 28,000-30,000 daltons as determined by sodium dodecyl sulfate polyacrylamide gel electrophoresis (SDS-PAGE). Under reducing conditions in SDS-PAGE the protein electrophoreses with a molecular weight of approximately 14,000 - 20,000 daltons. It is contemplated that these proteins are capable of stimulating promoting, or otherwise inducing cartilage and/or bone formation.

Human BMP-6 proteins of the invention are produced by culturing a cell transformed with a DNA sequence comprising nucleotide #160 to nucleotide #1698 as shown in Table III or a substantially similar sequence. BMP-6 proteins comprising amino acid #382 to amino acid #513 or a substantially similar sequence are recovered, isolated and purified from the culture medium.

The invention provides a family of BMP-7 proteins. Which includes purified human BMP-7 proteins, substantially free from other proteins with which they are co-produced. Human BMP-7 proteins are characterized by an amino acid sequence comprising amino acid #300 to amino acid #431 set forth in Table V. This amino acid sequence #300 to #431 is encoded by the DNA sequence of Table V from nucleotide #994 to #1389. BMP-7 proteins may be further characterized by an apparent molecular weight of 28,000-30,000 daltons as determined by sodium dodecyl sulfate polyacrylamide gel electrophoresis (SDS-PAGE). Under reducing conditions in SDS-PAGE the protein electrophoreses with a molecular weight of approximately 14,000 - 20,000 daltons. It is contemplated that these proteins are capable of stimulating, promoting, or otherwise inducing cartilage and/or bone formation.

Human BMP-7 proteins of the invention may be produced by culturing a cell transformed with a DNA sequence containing the nucleotide sequence the same or substantially the same as the nucleotide sequence shown in Table V comprising nucleotide # 97 to nucleotide #1389. BMP-7 proteins comprising the amino acid sequence the same or substantially the same as shown in Table V from amino acid #300 to amino acid #431 are recovered, isolated and purified from the culture medium.

The invention further provides a method wherein the proteins described above are utilized for obtaining related human protein/s or other mammalian cartilage and/or bone formation protein/s. Such methods are known to those skilled in the art of genetic engineering. One method for obtaining such proteins involves utilizing the human BMP-5, BMP-6 and BMP-7 coding sequences or portions thereof to design probes for screening human genomic and/or cDNA libraries to isolate human genomic and/or cDNA sequences. Additional methods within the art may employ the bovine and human BMP proteins of the invention to obtain other mammalian BMP cartilage and/or bone formation proteins.

Having identified the nucleotide sequences, the proteins are produced by culturing a cell transformed with the nucleotide sequence. This sequence or portions thereof hybridizes under stringent conditions to the nucleotide sequence of either BMP-5, BMP-6 or BMP-7 proteins and encodes a protein exhibiting cartilage and/or bone formation activity. The expressed protein is recovered and purified from the culture medium. The purified BMP proteins are substantially free from other proteinaceous materials with which they are co-produced, as well as from other contaminants.

BMP-5, BMP-6 and BMP-7 proteins may be characterized by the ability to promote, stimulate or otherwise induce the formation of cartilage and/or bone formation. It is further contemplated that the ability of these proteins to induce the formation of cartilage and/or bone may be exhibited by the ability to demonstrate cartilage and/or bone formation activity in the rat bone formation assay described below. It is further contemplated that the proteins of the invention demonstrate activity in this rat bone formation assay at a concentration of 10µg - 500µg/gram of bone formed. More particularly, it is contemplated these proteins may be characterized by the ability of 1µg of the protein to score at least +2 in the rat bone formation assay described below using either the original or modified scoring method.

Another aspect of the invention provides pharmaceutical compositions containing a therapeutically effective amount of a BMP-5, BMP-6 or BMP-7 protein in a pharmaceutically acceptable vehicle or carrier. Further compositions comprise at least one BMP-5, BMP-6 or BMP-7 protein. It is therefore contemplated that the compositions may contain more than one of the BMP proteins of the present invention as BMP-5, BMP-6 and BMP-7 proteins may act in concert with or perhaps synergistically with one another. The compositions of the invention are used to induce bone and/or cartilage formation. These compositions may also be used for wound healing and tissue repair.

Further compositions of the invention may include in addition to a BMP-5, BMP-6 or BMP-7 protein of the present invention at least one other therapeutically useful agent such as the proteins designated BMP-1, BMP-2 (also having been designated in the past as BMP-2A, BMP-2 Class I), BMP-3 and BMP-4 (also having been designated in the past as BMP-2B and BMP-2 Class II) disclosed in co-owned International Publication W088/00205 published 14 January 1988 and International Publication W089/10409 published 2 November 1989. Other therapeutically useful agents include growth factors such as epidermal growth factor (EGF), fibroblast growth factor (FGF), transforming growth factors (TGF-α and TGF-β), and platelet derived growth factor (PDGF).

The compositions of the invention may also include an appropriate matrix, for instance, for delivery and/or support of the composition and/or providing a surface for bone and/or cartilage formation. The matrix may proide solw release of the BMP protein and/or the appropriate environment for presentation of the BMP protein of the invention.

The compositions of the invention may be employed in methods for treating a number of bone and/or cartilage defects, and periodontal disease. They may also be employed in methods for treating various types of wounds and in tissue repair. These methods, according to the invention, entail administering a composition of the invention to a patient needing such bone and/or cartilage formation, wound healing or tissue repair. The method therefore involves administration of a therapeutically effective amount of a protein of the invention. These methods may also entail the administration of a protein of the invention in conjunction with at least one of the "BMP" proteins disclosed in the co-owned applications described above. In addition, these methods may also include the administration of a protein of the invention with other growth factors including EGF, FGF, TGF-α, TGF-β, and PDGF.

Still a further aspect of the invention are DNA sequences coding for expression of a protein of the invention. Such sequences include the sequence of nucleotides in a 5' to 3' direction illustrated in Tables III - V or DNA sequences which hybridize under stringent conditions with the DNA sequences of Tables III - V and encode a protein demonstrating ability to induce cartilage and/or bone formation. Such cartilage and/or bone formation may be demonstrated in the rat bone formation assay described below. It is contemplated that these proteins may demonstrate activity in this assay at a concentration of 10 µg - 500 µg/gram of bone formed. More particularly, it is contemplated that these proteins demonstrate the ability of 1µg of the protein to score at least +2 in the rat bone formation assay. Finally, allelic or other variations of the sequences of Tables III - V whether such nucleotide changes result in changes in the peptide sequence or not, are also included in the present invention.

A further aspect of the invention provides vectors containing a DNA sequence as described above in operative association with an expression control sequence therefor. These vectors may be employed in a novel process for producing a protein of the invention in which a cell line transformed with a DNA sequence directing expression of a protein of the invention in operative association with an expression control sequence therefor, is cultured in a suitable culture medium and a protein of the invention is recovered and purified therefrom. This claimed process may employ a number of known cells, both prokaryotic and eukaryotic, as host cells for expression of the polypeptide. The revovered BMP proteins are purified by isolating them from other proteinaceous materials with which they are co-produced as well as from other contaminants.

Other aspects and advantages of the present invention will be apparent upon consideration of the following detailed description and preferred embodiments thereof.

### Detailed Description of the Invention

Purified human BMP-5 proteins may be produced by culturing a host cell transformed with the DNA sequence of Table III. The expressed BMP-5 proteins are isolated and purified from the culture medium. Purified human BMP-5 proteins are expected to be characterized an amino acid sequence comprising amino acid #323 to #454 as shown in Table III. Purified BMP-5 human cartilage/bone proteins of the present invention are therefore produced by culturing a host cell transformed with a DNA sequence comprising nucleotide #699 to nucleotide #2060 as shown in Table III or substantially homologous sequences operatively linked to a heterologous regulatory control sequence and recovering and purifying from the culture medium a protein comprising the amino acid sequence as shown in Table III from amino acid #323 to amino acid #454 or a substantially homologous sequence.

In further embodiments the DNA sequence comprises the nucleotides encoding amino acids #323-#454. BMP-5 proteins may therefore be produced by culturing a host cell transformed with a DNA sequence comprising nucleotide #1665 to nucleotide #2060 as shown in Table III or substantially homologous sequences operatively linked to a heterologous regulatory control sequence and recovering and purifying from the culture medium a protein comprising amino acid #323 to amino acid #454 as shown in Table III or a substantially homologous sequence. The purified human BMP-5 proteins are substantially free from other proteinaceous materials with which they are co-produced, as well as from other contaminants.

Purified human BMP-6 proteins may be produced by culturing a host cell transformed with the DNA sequence of Table IV. The expressed proteins are isolated and purified from the culuture medium. Purified human BMP-6 proteins of the invention are expected to be characterized by an amino acid sequence comprising amino acid #382 to #513 as set forth in Table IV. These purified BMP-6 human cartilage/bone proteins of the present invention are therefore produced by culturing a host cell transformed with a DNA sequence comprising nucleotide #160 to nucleotide #1698 as set forth in Table IV or substantially homologous sequence operatively linked to a heterologous regulatory control sequence and recovering, isolating and purifying from the culture medium a protein comprising amino acid #382 to amino acid #513 as set forth in Table IV or a substantially homologous sequence.

Further embodiments may utilize the DNA sequence comrising the nucleotides encoding amino acids #382 - #513. Purified human BMP-6 proteins may therefore be produced by culturing a host cell transformed with the DNA sequence comprising nucleotide #1303 to #1698 as set forth in Table IV or substantially homologous sequences operatively linked to a heterologous regulatory control sequence and recovering and purifying from the culture medium a protein comprising amino acid #382 to #513 as set forth in Table IV or a substantially homologous sequence. The purified human BMP-6 proteins are substantially free from other proteinaceous materials with which they are co-produced, as well as from other contaminants.

Purified human BMP-7 proteins may be produced by culturing a host cell transformed with the DNA sequence of Table V. The expressed proteins are isolated and purified from the culture medium. Purified human BMP-7 proteins are expected to be characterized by an amino acid sequence comprising amino acid #300-#431 as shown in Table V. These purified BMP-7 human cartilage/bone proteins of the present invention are therefore produced by culturing a CHO cell transformed with a DNA sequence comprising nucleotide #97 to nucleotide #1389 as shown in Table V or substantially homologous sequences operatively linked to a heterologous regulatory control sequence and recovering, isolating and purifying from the culture medium a protein comprising the amino acid sequence as shown in Table V from amino acid #300 to amino acid #431 or a substantially homologous sequence.

Further emodiments may utilize the DNA sequence comprising the nucleotides encoding amino acids #300 - #431. Purified BMP-7 proteins may be produced by culturing a host cell transformed with a DNA comprising the DNA sequence as shown in Table V from nucleotide #994 - #1389 or substantially homologous sequences operatively linked to a heterologous regualtory control sequence and recovering, and purifying from the culture medium a protein comprising the amino acid sequence as shown in Table V from amino acid #300 to amino acid #431 or a substantially homologous sequence. The purified human BMP-7 proteins are substantially free from other proteinaceous materials from which they are co-produced, as well as from other contaminants.

BMP-5, BMP-6 and BMP-7 proteins are further characterized by the ability to demonstrate cartilage and/or bone formation activity. This activity may be demonstrated, for example, in the rat bone formation assay as described in Example III. It is further contemplated that these proteins demonstrate activity in the assay at a concentration of 10 µg - 500 1g/gram of bone formed. The proteins may be further characterized by the ability of 1µg to score at least +2 in this assay using either the original or modified scoring method descirbed further herein below.

BMP-5, BMP-6 and BMP-7 proteins may be further characterized by an apparent molecular weight of 28,000-30,000 daltons as determined by sodium dodecyl sulfate polyacrylamide gel electrophoresis (SDS-PAGE). Under reducing conditions in SDS-PAGE the protein electrophoresis with a molecular weight of approximately 14,000-20,000 daltons.

The proteins provided herein also include factors encoded by the sequences similar to those of Tables III - V but into which modifications are naturally provided (e.g. allelic variations in the nucleotide sequence which may result in amino acid changes in the polypeptide) or deliberately engineered. Similarly, synthetic polypeptides which wholly or partially duplicate continuous sequences of the amino acid residues of Tables III - V are encompassed by the invention. These sequences, by virtue of sharing primary, secondary, or tertiary structural and conformational characteristics with other cartilage/bone proteins of the invention may possess bone and/or cartilage growth factor biological properties in common therewith. Thus, they may be employed as biologically active substitutes for naturally-occurring proteins in therapeutic processes. Other specific mutations of the sequences of the proteins of the invention described herein involve modifications of a glycosylation site. These modification may involve O-linked or N-linked glycosylation sites. For instance, the absence of glycosylation or only partial glycosylation results from amino acid substitution or deletion at the asparagine-linked glycosylation recognition sites present in the sequences of the proteins of the invention, as shown in Table III - V. The asparagine-linked glycosylation recognition sites comprise tripeptide sequences which are specifically recognized by appropriate cellular glycosylation enzymes. These tripeptide sequences are either asparagine-X-threonine or asparagine-X-serine, where X is usually any amino acid. A variety of amino acid substitutions or deletions at one or both of the first or third amino acid positions of a glycosylation recognition site (and/or amino acid deletion at the second position) results in non-glycosylation at the modified tripeptide sequence. Expression of such altered nucleotide sequences produces variants which are not glycosylated at that site.

The present invention also encompasses the novel DNA sequences, free of association with DNA sequences encoding other proteinaceous materials, and coding on expression for the proteins of the invention. These DNA sequences include those depicted in Tables III - V in a 5' to 3' direction. Further included are those sequences which hybridize under stringent hybridization conditions [see, T. Maniatis et al, Molecular Cloning (A Laboratory Manual), Cold Spring Harbor Laboratory (1982), pages 387 to 389] to the DNA sequence of Tables III - V and demonstrate cartilage and/or bone formation activity in the rat bone formation assay. An example of one such stringent hybridization condition is hybridization at[6- 4 x SSC at 65°C, followed by a washing in 0.1 x SCC at 65°C for an hour. Alternatively, an exemplary stringent hybridization condition is in 50% formamide, 4 x SCC at 42°C.

Similarly, DNA sequences which encode proteins similar to the protein encoded by the sequences of Tables III - V, but which differ in codon sequence due to the degeneracies of the genetic code or allelic variations (naturally-occurring base changes in the species population which may or may not result in an amino acid change) also encode the proteins of the invention described herein. Variations in the DNA sequences of Tables III - V which are caused by point mutations or by induced modifications (including insertion, deletion, and substitution) to enhance the activity, half-life or production of the polypeptides encoded thereby are also encompassed in the invention.

In a further aspect, the invention provides a method for obtaining related human proteins or other mammalian BMP-5, BMP-6 and BMP-7 proteins. One method for obtaining such proteins entails, for instance, utilizing the human BMP-5, BMP-6 and BMP-7 coding sequence disclosed herein to probe a human genomic library using standard techniques for the human gene or fragments thereof. Sequences thus identified may also be used as probes to identify a human cell line or tissue which synthesizes the analogous cartilage/bone protein. A cDNA library is synthesized and screened with probes derived from the human or bovine coding sequences. The human sequence thus identified is transformed into a host cell, the host cell is cultured and the protein recovered, isolated and purified from the culture medium. The purified protein is predicted to exhibit cartilage and/or bone formation activity in the rat bone formation assay of Example III.

Another aspect of the present invention provides a novel method for producing the BMP-5, BMP-6 and BMP-7 proteins of the invention. The method of the present invention involves culturing a suitable cell or cell line, which has been transformed with a DNA sequence as described above coding for expression of a protein of the invention, under the control of known regulatory sequences. Regulatory sequences include promoter fragments, terminator fragments and other suitable sequences which direct the expression of the protein in an appropriate host cell. Methods for culturing suitable cell lines are within the skill of the art. The transformed cells are cultured and the BMP proteins expressed thereby are recovered, isolated and purified from the culture medium using purification techniques known to those skilled in the art. The purified BMP proteins are substantially free from other proteinaceous materials with which they are co-produced, as well as other contaminants. Purified BMP proteins of the invention are substantially free from materials with which the proteins of the invention exist in nature.

Suitable cells or cell lines may be mammalian cells, such as Chinese hamster ovary cells (CHO). The selection of suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U.S. Patent 4,419,446. Other suitable mammalian cell lines include but are not limited to the monkey COS-1 cell line and the CV-1 cell line.

Bacterial cells may also be suitable hosts. For example, the various strains of E. coli (e.g., HB101, MC1061) are well-known as host cells in the field of biotechnology. Various strains of B. subtilis, Pseudomonas, other bacilli and the like may also be employed in this method.

Many strains of yeast cells known to those skilled in the art may also be available as host cells for expression of the polypeptides of the present invention. Additionally, where desired, insect cells may be utilized as host cells in the method of the present invention. See, e.g. Miller et al, Genetic Engineering, 8:277-298 (Plenum Press 1986) and references cited therein.

Another aspect of the present invention provides vectors for use in the method of expression of the proteins of the invention. The vectors contain the novel DNA sequences which code for the BMP-5, BMP-6 and BMP-7 proteins of the invention. Additionally, the vectors also contain appropriate expression control sequences permitting expression of the protein sequences. Alternatively, vectors incorporating truncated or modified sequences as described above are also embodiments of the present invention and useful in the production of the proteins of the invention. The vectors may be employed in the method of transforming cell lines and contain selected regulatory sequences in operative association with the DNA coding sequences of the invention which are capable of directing the replication and expression thereof in selected host cells. Useful regulatory sequences for such vectors are known to those skilled in the art and may be selected depending upon the selected host cells. Such selection is routine and does not form part of the present invention. Host cells transformed with such vectors and progeny thereof for use in producing BMP-5, BMP-6 and BMP-7 proteins are also provided by the invention.

One skilled in the art can construct mammalian expression vectors by employing the DNA sequences of the invention and known vectors, such as pCD [Okayama et al., Mol. Cell Biol., 2:161-170 (1982)] and pJL3, pJL4 [Gough et al., EMBO J., 4:645-653 (1985)]. Similarly, one skilled in the art could manipulate the sequences of the invention by eliminating or replacing the mammalian regulatory sequences flanking the coding sequence with bacterial sequences to create bacterial vectors for intracellular or extracellular expression by bacterial cells. For example, the coding sequences could be further manipulated (e.g. ligated to other known linkers or modified by deleting non-coding sequences there-from or altering nucleotides therein by other known techniques). The modified coding sequence could then be inserted into a known bacterial vector using procedures such as described in T. Taniguchi et al., Proc. Natl Acad. Sci. USA, 77:5230-5233 (1980). This exemplary bacterial vector could then be transformed into bacterial host cells and a protein of the invention expressed thereby. For a strategy for producing extracellular expression of a cartilage and/or bone protein of the invention in bacterial cells., see, e.g. European patent application EPA 177,343.

Similar manipulations can be performed for the construction of an insect vector [See, e.g. procedures described in published European patent application 155,476] for expression in insect cells. A yeast vector could also be constructed employing yeast regulatory sequences for intracellular or extracellular expression of the factors of the present invention by yeast cells. [See, e.g., procedures described in published PCT application W086/00639 and European patent application EPA 123,289].

A method for producing high levels of a protein of the invention from mammalian cells involves the construction of cells containing multiple copies of the heterologous gene encoding proteins of the invention. The heterologous gene may be linked to an amplifiable marker, e.g. the dihydrofolate reductase (DHFR) gene for which cells containing increased gene copies can be selected for propagation in increasing concentrations of methotrexate (MTX) according to the procedures of Kaufman and Sharp, J. Mol. Biol., 159:601-629 (1982). This approach can be employed with a number of different cell types.

For instance, a plasmid containing a DNA sequence for a protein of the invention in operative association with other plasmid sequences enabling expression thereof and the DHFR expression plasmid pAdA26SV(A)3 [Kaufman and Sharp, Mol. Cell. Biol., 2:1304 (1982)] may be co-introduced into DHFR-deficient CHO cells, DUKX-BII, by calcium phosphate coprecipitation and transfection, electroperation or protoplast fusion. DHFR expressing transformants are selected for growth in alpha media with dialyzed fetal calf serum, and subsequently selected for amplification by growth in increasing concentrations of MTX (sequential steps in 0.02, 0.2, 1.0 and 5uM MTX) as described in Kaufman et al., Mol Cell Biol., 5:1750 (1983). Protein expression should increase with increasing levels of MTX resistance.

Transformants are cloned, and the proteins of the invention are recovered, isolated, and purified from the culture medium. Characterization of expressed proteins may be carried out using standard techniques. For instance, characterization may include pulse labeling with [35^{S}] methionine or cysteine, or polyacrylamide gel electrphoresis. Biologically active protein expression is monitored by the Rosen-modified Sampath - Reddi rat bone formation assay described above in Example III. Similar procedures can be followed to produce other related proteins.

A protein of the present invention, which induces cartilage and/or bone formation in circumstances where bone and/or cartilage is not normally formed, has application in the healing of bone fractures and cartilage defects in humans and other animals. A preparation employing a protein of the invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery. A protein of the invention may be used in the treatment of periodontal disease, and in other tooth repair processes. Such agents may provide an environment to attract bone-forming cells, stimulate growth of bone-forming cells or induce differentiation of progenitors of bone-forming cells. A variety of osteogenic, cartilage-inducing and bone inducing factors have been described. See, e.g. European Patent Applications 148,155 and 169,016 for discussions thereof.

The proteins of the invention may also be used in wound healing and related tissue repair. The types of wounds include, but are not limited to burns, incisions and ulcers. See, e.g. PCT Publication W084/01106 for discussion of wound healing and related tissue repair.

A further aspect of the invention includes pharmaceutical compositions, which can preferably be used for repairing fractures and other conditions related to bone and/or cartilage defects or periodontal diseases. In addition, the invention comprises pharmaceutical compositions for wound healing and tissue repair. Such compositions comprise a therapeutically effective amount of at least one of the BMP proteins BMP-5, BMP-6 and BMP-7 of the invention in admixture with a pharmaceutically acceptable vehicle, carrier or matrix.

It is expected that the proteins of the invention may act in concert with or perhaps synergistically with one another or with other related proteins and growth factors. Therapeutic methods and compositions of the invention therefore comprise one or more of the proteins of the present invention. Further therapeutic methods and compositions of the invention therefore comprise a therapeutic amount of at least one protein of the invention with a therapeutic amount of at least one of the other "BMP" proteins BMP-1, BMP-2, BMP-3 and BMP-4 disclosed in co-owned Published International Applications WO88/00205 and WO89/10409 as mentioned above. Such methods and compositions of the invention may comprise proteins of the invention or portions thereof in combination with the above-mentioned "BMP" proteins or portions thereof.

Such combination may comprise individual separate molecules of the proteins or heteromolecules such as heterodimers formed by portions of the respective proteins. For example, a method and composition of the invention may comprise a BMP protein of the present invention or a portion thereof linked with a portion of another "BMP" protein to form a heteromolecule.

Further pharmaceutical compositions of the invention comprise the proteins of the invention or portions thereof in combination with other agents beneficial to the treatment of the bone and/or cartilage defect, wound, or tissue in question. These agents include various growth factors such as epidermal growth factor (EGF), fibroblast growth factor (FGF), platelet derived growth factor (PDGF), transforming growth factors (TGF-α and TGF-β), K-fibroblast growth factor (kFGF), parathyroid hormone (PTH), leukemia inhibitory factor (LIF/HILDA, DIA) and insulin-like growth factor (IGF-I and IGF-II). Portions of these agents may also be used in compositions of the invention.

The preparation and formulation of such physiologically acceptable protein compositions, having due regard to pH, isotonicity, stability and the like, is within the skill of the art. The therapeutic compositions are also presently valuable for veterinary applications due to the apparent lack of species specificity in cartilage and bone growth factor proteins. Domestic animals and thoroughbred horses in addition to humans are desired patients for such treatment with the proteins of the present invention.

The composition can be administered topically, systemically, or locally as an implant or device. When administered, the therapeutic composition for use in this invention is, of course, in a pyrogen-free, physiologically acceptable form. Further, the composition may desirably be encapsulated or injected in a viscous form for delivery to the site of cartilage and/or bone or tissue damage. Topical administration may be suitable for wound healing and tissue repair.

Preferably for bone and/or cartilage formation, the composition would include a matrix capable of delivering the BMP proteins of the invention to the site of bone and/or cartilage damage, providing a structure for the developing bone and cartilage and optimally capable of being resorbed into the body. The matrix may provide slow release of the BMP proteins or other factors comprising the composition. Such matrices may be formed of materials presently in use for other implanted medical applications.

The choice of matrix material is based on biocompatibility, biodegradability, mechanical properties, cosmetic appearance and interface properties. The particular application of the compositions of the invention will define the appropriate formulation. Potential matrices for the compositions may be biodegradable and chemically defined calcium sulfate, tricalciumphosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are nonbiodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalciumphosphate. The bioceramics may be altered in composition, such as in calcium-aluminate-phosphate and processing to alter pore size, particle size, particle shape, and biodegradability.

The dosage regimen will be determined by the attending physician considering various factors which modify the action of the proteins of the invention. Factors which may modify the action of the proteins of the invention include the amount of bone weight desired to be formed, the site of bone damage, the condition of the damaged bone, the size of a wound, type of damaged tissue, the patient's age, sex, and diet, the severity of any infection, time of administration and other clinical factors. The dosage may vary with the type of matrix used in the reconstitution and the type or types of bone and/or cartilage proteins present in the composition. The addition of other known growth factors, such as EGF, PDGF, TGF-α, TGF-β, and IGF-I and IGF-II to the final composition, may also effect the dosage.

Progress can be monitored by periodic assessment of cartilage and/or bone growth and/or repair. The progress can be monitored, for example, using x-rays, histomorphometric determinations and tetracycline labeling.

The following examples illustrate practice of the present invention in recovering and characterizing bovine cartilage and/or bone proteins of the invention and employing these proteins to recover the corresponding human protein or proteins and in expressing the proteins via recombinant techniques.

### EXAMPLE I

### Isolation of Bovine Cartilaqe/Bone Inductive Protein

Ground bovine bone powder (20-120 mesh, Helitrex) is prepared according to the procedures of M. R. Urist et al., Proc. Natl Acad. Sci USA, 70:3511 (1973) with elimination of some extraction steps as identified below. Ten kgs of the ground powder is demineralized in successive changes of 0.6N HCl at 4°C over a 48 hour period with vigorous stirring. The resulting suspension is extracted for 16 hours at 4°C with 50 liters of 2M CaCl₂ and 10mM ethylenediamine-tetraacetic acid [EDTA], and followed by extraction for 4 hours in 50 liters of 0.5M EDTA. The residue is washed three times with distilled water before its resuspension in 20 liters of 4M guanidine hydrochloride [GuCl], 20mM Tris (pH 7.4), 1mM N-ethylmaleimide, 1mM iodoacetamide, 1mM phenylmethylsulfonyl fluorine as described in Clin. Orthop. Rel. Res., 171: 213 (1982). After 16 to 20 hours the supernatant is removed and replaced with another 10 liters of GuCl buffer. The residue is extracted for another 24 hours.

The crude GuCl extracts are combined, concentrated approximately 20 times on a Pellicon apparatus with a 10,000 molecular weight cut-off membrane, and then dialyzed in 50nM Tris, 0.1M NaCl, 6M urea (pH7.2), the starting buffer for the first column. After extensive dialysis the protein is loaded on a 4 liter DEAE cellulose column and the unbound fractions are collected.

The unbound fractions are concentrated and dialyzed against 50mM NaAc, 50mM NaCl (pH 4.6) in 6M urea. The unbound fractions are applied to a carboxymethyl cellulose column. Protein not bound to the column is removed by extensive washing with starting buffer, and the material containing protein having bone and/or cartilage formation activity as measured by the Rosen-modified Sampath-Reddi assay (described in Example III below) desorbed from the column by 50mM NaAc, 0.25mM NaCl, 6M urea (pH 4.6). The protein from this step elution is concentrated 20- to 40- fold, then diluted 5 times with 80mM KPO₄, 6M urea (pH6.0). The pH of the solution is adjusted to 6.0 with 500mM K₂HPO₄. The sample is applied to an hydroxylapatite column (LKB) equilibrated in 80mM KPO₄, 6M urea (pH6.0) and all unbound protein is removed by washing the column with the same buffer. Protein having bone and/or cartilage formation activity is eluted with 100mM KPO₄ (pH7.4) and 6M urea.

The protein is concentrated approximately 10 times, and solid NaCl added to a final concentration of 0.15M. This material is applied to a heparin - Sepharose column equilibrated in 50mM KPO₄, 150mM NaCl, 6M urea (pH7.4). After extensive washing of the column with starting buffer, a protein with bone and/or cartilage inductive activity is eluted by 50mM KPO₄, 700mM NaCl, 6M urea (pH7.4). This fraction is concentrated to a minimum volume, and 0.4ml aliquots are applied to Superose 6 and Superose 12 columns connected in series, equilibrated with 4M GuCl, 20mM Tris (pH7.2) and the columns developed at a flow rate of 0.25ml/min. The protein demonstrating bone and/or cartilage inductive activity corresponds to an approximate 30,000 dalton protein.

The above fractions from the superose columns are pooled, dialyzed against 50mM NaAc, 6M urea (pH4.6), and applied to a Pharmacia MonoS HR column. The column is developed with a gradient to 1.0M NaCl, 50mM NaAc, 6M urea (pH4.6). Active bone and/or cartilage formation fractions are pooled. The material is applied to a 0.46 x 25cm Vydac C4 column in 0.1% TFA and the column developed with a gradient to 90% acetonitrile, 0.1% TFA (31.5% acetonitrile, 0.1% TFA to 49.5% acetonitrile, 0.1% TFA in 60 minutes at lml per minute). Active material is eluted at approximately 40-44% acetonitrile. Fractions were assayed for cartilage and/or bone formation activity.The active material is further fractionated on a MonoQ column. The protein is dialyzed against 6M urea, 25mM diethanolamine, pH 8.6 and then applied to a 0.5 by 5 cm MonoQ column (Pharmacia) which is developed with a gradient of 6M urea, 25mM diethanolamine, pH 8.6 and 0.5 M NaCl, 6M urea, 25mM diethanolamine, pH 8.6. Fractions are brought to pH3.0 with 10% trifluoroacetic acid (TFA). Aliquots of the appropriate fractions are iodinated by one of the following methods: P. J. McConahey et al, Int. Arch. Allergy, 29:185-189 (1966); A. E. Bolton et al, Biochem J., 133:529 (1973); and D. F. Bowen-Pope, J. Biol. Chem., 237:5161 (1982). The iodinated proteins present in these fractions are analyzed by SDS gel electrophoresis.

### EXAMPLE II

### Characterization of Bovine Cartilage/Bone Inductive Factor

### A. Molecular Weight

Approximately 5µg protein from Example I in 6M urea, 25mM diethanolamine, pH 8.6, approximately 0.3 M NaCl is made 0.1% with respect to SDS and dialyzed against 50 mM tris/HCl 0.1% SDS pH 7.5 for 16 hrs. The dialyzed material is then electrophorectically concentrated against a dialysis membrane [Hunkapillar et al Meth. Enzymol. 91: 227-236 (1983)] with a small amount of I 125 labelled counterpart. This material (volume approximately 100µl) is loaded onto a 12% polyacrylamide gel and subjected to SDS-PAGE [Laemmli, U.K. Nature, 227:680-685 (1970)] without reducing the sample with dithiothreitol. The molecular weight is determined relative to prestained molecular weight standards (Bethesda Research Labs). Following autoradiography of the unfixed gel the approximate 28,000-30,000 dalton band is excised and the protein electrophoretically eluted from the gel (Hunkapillar et al supra). Based on similar purified bone fractions as described in the co-pending "BMP" applications described above wherein bone and/or cartilage activity is found in the 28,000-30,000 region, it is inferred that this band comprises bone and/or cartilage inductive fractions.

### B. Subunit Characterization

The subunit composition of the isolated bovine bone protein is also determined. The eluted protein described above is fully reduced and alkylated in 2% SDS using iodoacetate and standard procedures and reconcentrated by electrophoretic packing. The fully reduced and alkylated sample is then further submitted to SDS-PAGE on a 12% gel and the resulting approximate 14,000-20,000 dalton region having a doublet appearance located by autoradiography of the unfixed gel. A faint band remains at the 28,000-30,000 region. Thus the 28,000-30,000 dalton protein yields a broad region of 14,000-20,000 which may otherwise also be interpreted and described as comprising two broad bands of approximately 14,000-16,000 and 16,000-20,000 daltons.

### EXAMPLE III

### Rosen Modified Sampath-Reddi Assay

A modified version of the rat bone formation assay described in Sampath and Reddi, Proc. Natl. Acad. Sci. U.S.A., 80:6591-6595 (1983) is used to evaluate bone and/or cartilage activity of the proteins of the invention. This modified assay is herein called the Rosen-modified Sampath-Reddi assay. The ethanol precipitation step of the Sampath-Reddi procedure is replaced by dialyzing (if the composition is a solution) or diafiltering (if the composition is a suspension) the fraction to be assayed against water. The solution or suspension is then redissolved in 0.1 % TFA, and the resulting solution added to 20mg of rat matrix. A mock rat matrix sample not treated with the protein serves as a control. This material is frozen and lyophilized and the resulting powder enclosed in #5 gelatin capsules. The capsules are implanted subcutaneously in the abdominal thoracic area of 21 - 49 day old male Long Evans rats. The implants are removed after 7 - 14 days. Half of each implant is used for alkaline phosphatase analysis [See, A. H. Reddi et al., Proc. Natl Acad Sci., 69:1601 (1972)].

The other half of each implant is fixed and processed for histological analysis. Glycolmethacrylate sections (1µm) are stained with Von Kossa and acid fuschin or toluidine blue to score the amount of induced bone and cartilage formation present in each implant. The terms +1 through +5 represent the area of each histological section of an implant occupied by new bone and/or cartilage cells and newly formed bone and matrix. Two scoring methods are herein described. In the first scoring method a score of +5 indicates that greater than 50% of the implant is new bone and/or cartilage produced as a direct result of protein in the implant. A score of +4, +3, +2 and +1 would indicate that greater than 40%, 30%, 20% and 10% respectively of the implant contains new cartilage and/or bone. The second scoring method (which hereinafter may be referred to as the modified scoring method) is as follows: three non-adjacent sections are evaluated from each implant and averaged. "+/-" indicates tentative identification of cartilage or bone; "+1" indicates >10% of each section being new cartilage or bone; "+2", >25%; "+3", >50%; "+4", -75%; "+5", >80%. The scores of the individual implants are tabulated to indicate assay variability.

It is contemplated that the dose response nature of the cartilage and/or bone inductive protein containing samples of the matrix samples will demonstrate that the amount of bone and/or cartilage formed increases with the amount of cartilage/bone inductive protein in the sample. It is contemplated that the control samples will not result in any bone and/or cartilage formation.

As with other cartilage and/or bone inductive proteins such as the above-mentioned "BMP" proteins, the bone and/or cartilage formed is expected to be physically confined to the space occupied by the matrix. Samples are also analyzed by SDS gel electrophoresis and isoelectric focusing followed by autoradiography. The activity is correlated with the protein bands and pI. To estimate the purity of the protein in a particular fraction an extinction coefficient of 1 OD/mg-cm is used as an estimate for protein and the protein is run on SDS-PAGE followed by silver staining or radioiodination and autoradiography.

### EXAMPLE IV

### A. Bovine Protein Composition

The gel slice of the approximate 14,000-20,000 dalton region described in Example IIB is fixed with methanol-acetic acid-water using standard procedures, briefly rinsed with water, then neutralized with 0.1M ammonium bicarbonate. Following dicing the gel slice with a razor blade, the protein is digested from the gel matrix by adding 0.2 pg of TPCK-treated trypsin (Worthington) and incubating the gel for 16 hr. at 37 degrees centigrade. The resultant digest is then subjected to RPHPLC using a C4 Vydac RPHPLC column and 0.1% TFA-water 0.1% TFA water-acetonitrile gradient. The resultant peptide peaks were monitored by UV absorbance at 214 and 280 nm and subjected to direct amino terminal amino acid sequence analysis using an Applied Biosystems gas phase sequenator (Model 470A). One tryptic fragment is isolated by standard procedures having the following amino acid sequence as represented by the amino acid standard three-letter symbols and where "Xaa" indicates an unknown amino acid the amino acid in parentheses indicates uncertainty in the sequence:

Xaa-His-Glu-Leu-Tyr-Val-Ser-Phe-(Ser)

The following four oligonucleotide probes are designed on the basis of the amino acid sequence of the above-identified tryptic fragment and synthesized on an automated DNA synthesizer.

PROBE #1: GTRCTYGANATRCANTC
PROBE #2: GTRCTYGANATRCANAG

PROBE #3: GTRCTYAAYATRCANTC
PROBE #4: GTRCTYAAYATRCANAG

The standard nucleotide symbols in the above identified probes are as follows: A,adenosine; C,cytosine; G,guanine; T,thymine; N, adenosine or cytosine or guanine or thymine; R,adenosine or guanine; and Y,cytosine or thymine.

Each of the probes consists of pools of oligonucleotides. Because the genetic code is degenerate (more than one codon can code for the same amino acid), a mixture of oligonucleotides is synthesized that contains all possible nucleotide sequences encoding the amino acid sequence of the tryptic. These probes are radioactively labeled and employed to screen a bovine cDNA library as described below.

### B. Bovine BMP-5

Poly(A) containing RNA is isolated by oligo(dT) cellulose chromatography from total RNA isolated from fetal bovine bone cells by the method of Gehron-Robey et al in Current Advances in Skeletogenesis, Elsevier Science Publishers (1985). The total RNA was obtained from Dr. Marion Young, National Institute of Dental Research, National Institutes of Health. A cDNA library is made in lambda gt10 (Toole et al supra) and plated on 50 plates at 8000 recombinants per plate. These recombinants (400,000) are screened on duplicate nitrocellulose filters with a combination of Probes 1, 2, 3, and 4 using the Tetramethylammonium chloride (TMAC) hybridization procedure [see Wozney et al Science, 242: 1528-1534 (1988)]. Twenty-eight positives are obtained and are replated for secondaries. Duplicate nitrocellulose replicas again are made. One set of filters are screened with Probes #1 and #2; the other with Probes #3 and #4. Six positives are obtained on the former, 21 positives with the latter. One of the six, called HEL5, is plague purified, a phage plate stock made, and bacteriophage DNA isolated. This DNA is digested with EcoRI and subcloned into M13 and pSP65 (Promega Biotec, Madison, Wisconsin) [Melton, et al. Nucl. Acids Res. 12: 7035-7056 (1984)]. The DNA sequence and derived amino acid sequence of this fragment is shown in Table I.

DNA sequence analysis of this fragment in M13 indicates that it encodes the desired tryptic peptide sequence set forth above, and this derived amino acid sequence is preceded by a basic residue (Lys) as predicted by the specificity of trypsin. The underlined portion of the sequence in Table I from amino acid #42 to #48 corresponds to the tryptic fragment identified above from which the oligonucleotide probes are designed. The derived amino acid sequence Ser-GIy-Ser-His-Gln-Asp-Ser-Ser-Arg as set forth in Table I from amino acid #15 to #23 is noted to be similar to a tryptic fragment sequence Ser-Thr-Pro-Ala-Gln-Asp-Val-Ser-Arg found in the 28,000 - 30,000 dalton purified bone preparation as described in the "BMP" Publications W088/00205 and W089/10409 mentioned above. This fragment set forth in Table I is a portion of the DNA sequence which encodes a bovine BMP-5 protein. The DNA sequence shown in Table I indicates an open reading frame from the 5' end of the clone of 420 base pairs, encoding a partial peptide of 140 amino acid residues (the first 7 nucleotides are of the adaptors used in the cloning procedure). An in-frame stop codon (TAA) indicates that this clone encodes the carboxy-terminal part of bovine BMP-5.

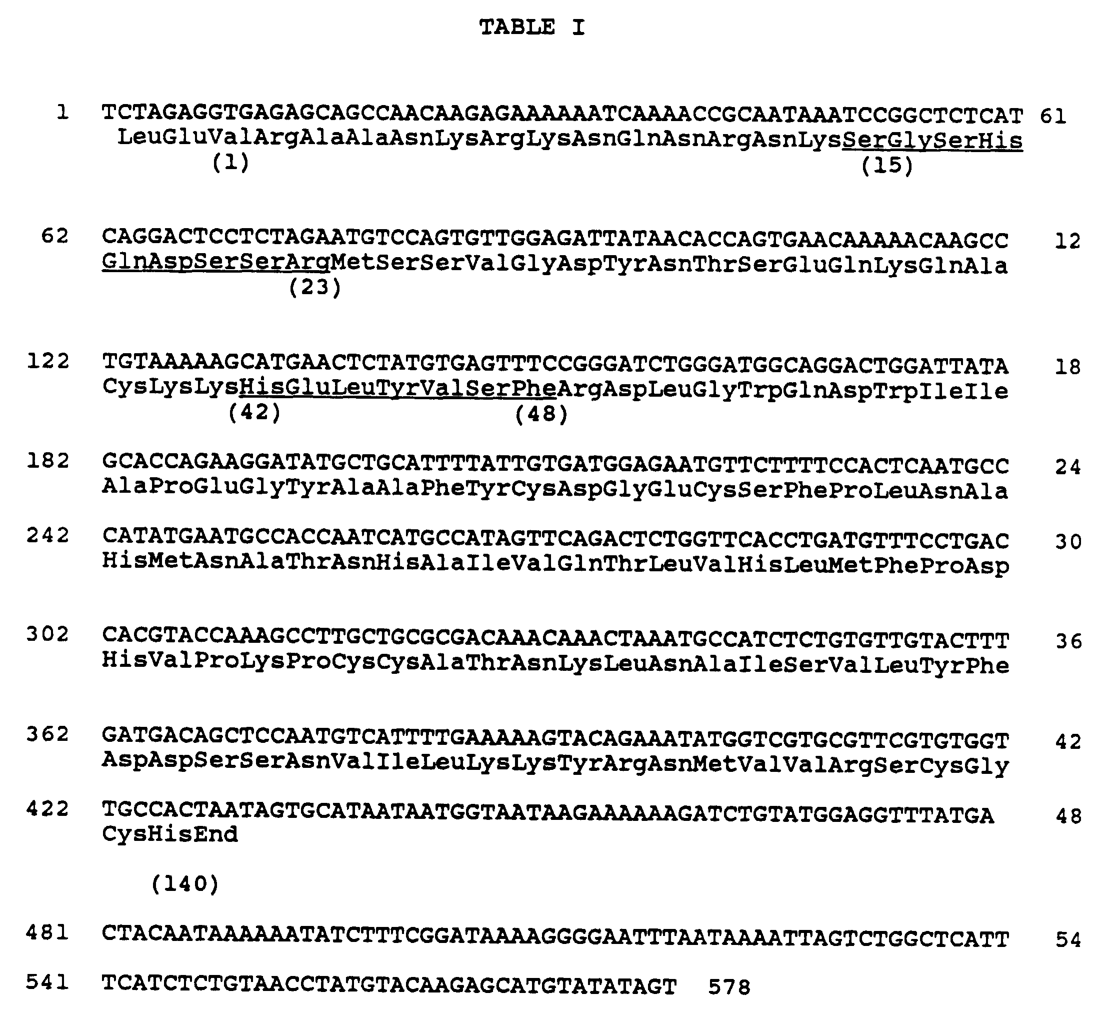

### C. Bovine BMP-6

The remaining positive clones (the second set containing 21 positives) isolated with Probes #1, #2, #3, and #4 described above are screened with HEL5 and a further clone is identified that hybridizes under reduced hybridization conditions [5x SSC, 0.1% SDS, 5X Denhardt's, 100 µg/ml salmon sperm DNA standard hybridization buffer (SHB) at 65°C, wash in 2XSSC 0.1% SDS at 65°C]. This clone is plaque purified, a phage plate stock made and bacteriophage DNA isolated. The DNA sequence and derived amino acid sequence of a portion of this clone is shown in Table II. This sequence represents a portion of the DNA sequence encoding a bovine BMP-6 cartilage/bone protein of the invention.

The first underlined portion of the sequence in Table II from amino acid #97 - amino acid #105 corresponds to the tryptic fragment found in the 28,000-30,000 dalton purified bovine bone preparation (and its reduced form at approximately 18,000-20,000 dalton reduced form) as described in the "BMP" Publications W088/00205 and W089/10409 mentioned above. The second underlined sequence in Table II from amino acid #124 - amino acid #130 corresponds to the tryptic fragment identified above from which the oligonucleotide probes are designed.

The DNA sequence of Table II indicates an open reading frame of 666 base pairs starting from the 5' end of the sequence of Table II, encoding a partial peptide of 222 amino acid residues. An in-frame stop codon (TGA) indicates that this clone encodes the carboxy-terminal part of a bovine BMP-6 protein. Based on knowledge of other BMP proteins and other proteins in the TGF-β family, it is predicted that the precursor polypeptide would be cleaved at the three basic residues (ArgArgArg) to yield a mature peptide beginning with residue 90 or 91 of the sequence of Table II.

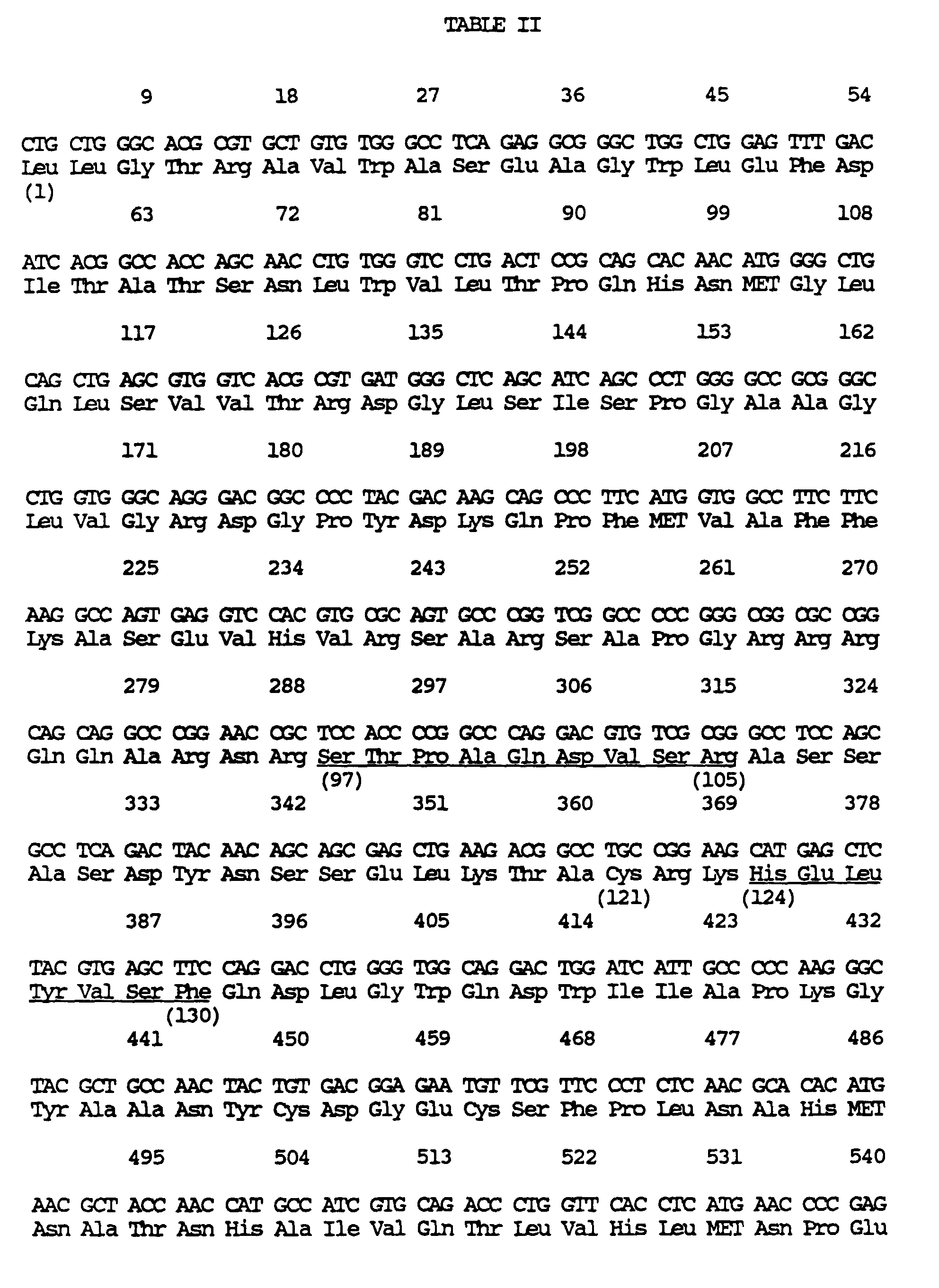

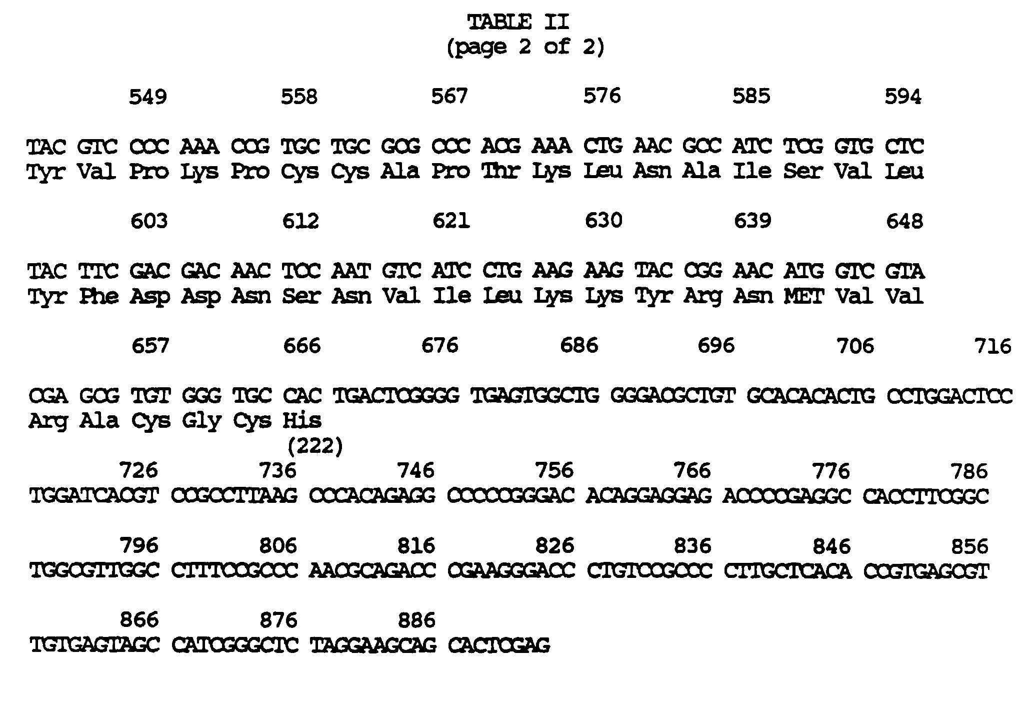

### EXAMPLE V

### A. Human Protein Composition

Human cell lines which synthesize BMP-5 and/or BMP-6 mRNAs are identified in the following manner. RNA is isolated from a variety of human cell lines, selected for poly(A)-containing RNA by chromatography on oligo(dT) cellulose, electrophoresed on a formaldehyde-agarose gel, and transferred to nitrocellulose. A nitrocellulose replica of the gel is hybridized to a single stranded M13 ³²P-labeled probe corresponding to the above mentioned BMP-5 EcoRI-BglII fragment containing nucleotides 1-465 of the sequence of Table I. A strongly hybridizing band is detected in the lane corresponding to the human osteosarcoma cell line U-20S RNA. Another nitrocellulose replica is hybridized to a single stranded M13 ³²P-labeled probe containing the PstI-SmaI fragment of bovine BMP-6 (corresponding to nucleotides 106-261 of Table II). It is found that several RNA species in the lane corresponding to U-20S RNA hybridize to this probe.

A cDNA Library is made in the vector lambda ZAP (Stratagene) from U-20S poly(A)-containing RNA using established techniques (Toole et al.). 750,000 recombinants of this library are plated and duplicate nitrocellulose replicas made. The SmaI fragment of bovine BMP-6 corresponding to nucleotides 259-751 of Table II is labeled by nick-translation and hybridized to both sets of filters in SHB at 65°C. One set of filters is washed under stringent conditions (0.2X SSC, 0.1% SDS at 65°C), the other under reduced stringency conditions (1X SSC, 0.1% SDS at 65°C). Many duplicate hybridizing recombinants (approximately 162) are noted. 24 are picked and replated for secondaries. Three nitrocellulose replicas are made of each plate. One is hybridized to the BMP-6 SmaI probe, one to a nick-translated BMP-6 PstI-SacI fragment (nucleotides 106-378 of Table II), and the third to the nick-translated BMP-5 XbaI fragments (nucleotides 1-76 of Table I). Hybridization and washes are carried out under stringent conditions.

### B. Human BMP-5 Proteins

17 clones that hybridize to the third probe more strongly than to the second probe are plaque purified. DNA sequence analysis of one of these, U2-16, indicates that it encodes human BMP-5. U2-16 was deposited with the American Type Culture Collection (ATCC), Rockville, Maryland on June 22, 1989 under accession number ATCC 68109. This deposit as well as the other deposits described herein are made under the provisions of the Budapest Treaty on the International Recognition of the Deposit of Microorganisms for the Purposes of Patent Procedure and the Regulations thereunder (Budapest Treaty). U2-16 contains an insert of approximately 2.1 Kb. The DNA sequence and derived amino acid sequence of U2-16 is shown below in Table III. This clone is expected to contain all of the nucleotide sequence necessary to encode human BMP-5 proteins. The cDNA sequence of Table III contains an open reading frame of 1362 bp, encoding a protein of 454 amino acids, preceded by a 5' untranslated region of 700 bp with stop codons in all frames, and contains a 3' untranslated region of 90 bp following the in frame stop codon (TAA).

This protein of 454 amino acids has a molecular weight of approximately 52,000 daltons as predicted by its amino acid sequence, and is contemplated to represent the primary translation product. Based on knowledge of other BMP proteins and other proteins within the TGF-β family, it is predicted that the precursor polypeptide would be cleaved at the tribasic peptide Lys Arg Lys yielding a 132 amino acid mature peptide beginning with amino acid #323 "Asn". The processing of BMP-5 into the mature form is expected to involve dimerization and removal of the N-terminal region in a manner analogous to the processing of the related protein TGF-β [L.E. Gentry, et al., Molec. & Cell. Biol. 8:4162 (1988); R. Dernyck, et al., Nature 316:701(1985)].

It is contemplated therefore that the mature active species of BMP-5 comprises a homodimer of 2 polypeptide subunits each subunit comprising amino acid #323 - #454 with a predicted molecular weight of approximately 15,000 daltons. Further active BMP-5 species are contemplated, for example, proprotein dimers or proprotein subunits linked to mature subunits. Additional active species may comprise amino acid #329 - #454 such species including homologous the tryptic sequences found in the purified bovine material. Also contemplated are BMP-5 proteins comprising amino acids #353-#454 thereby including the first conserved cysteine residue.

The underlined sequence of Table III from amino acid #329 to #337 Ser-Ser-Ser-His-Gln-Asp-Ser-Ser-Arg shares homology with the bovine sequence of Table I from amino acid #15 to #23 as discussed above in Example IV. Each of these sequences shares homology with a tryptic fragment sequence Ser-Thr-Pro-Ala-Gln-Asp-Val-Ser-Arg found in the 28,000 - 30,000 dalton purified bone preparation (and its reduced form at approximately 18,000 - 20,000 daltons) as described in the "BMP" published applications WO88/00205 and WO89/10409 mentioned above.

The underlined sequence of Table III from amino acid #356 to #362 His-Glu-Leu-Tyr-Val-Ser-Phe corresponds to the tryptic fragment identified in the bovine bone preparation described above from which the oligonucleotide probes are designed.

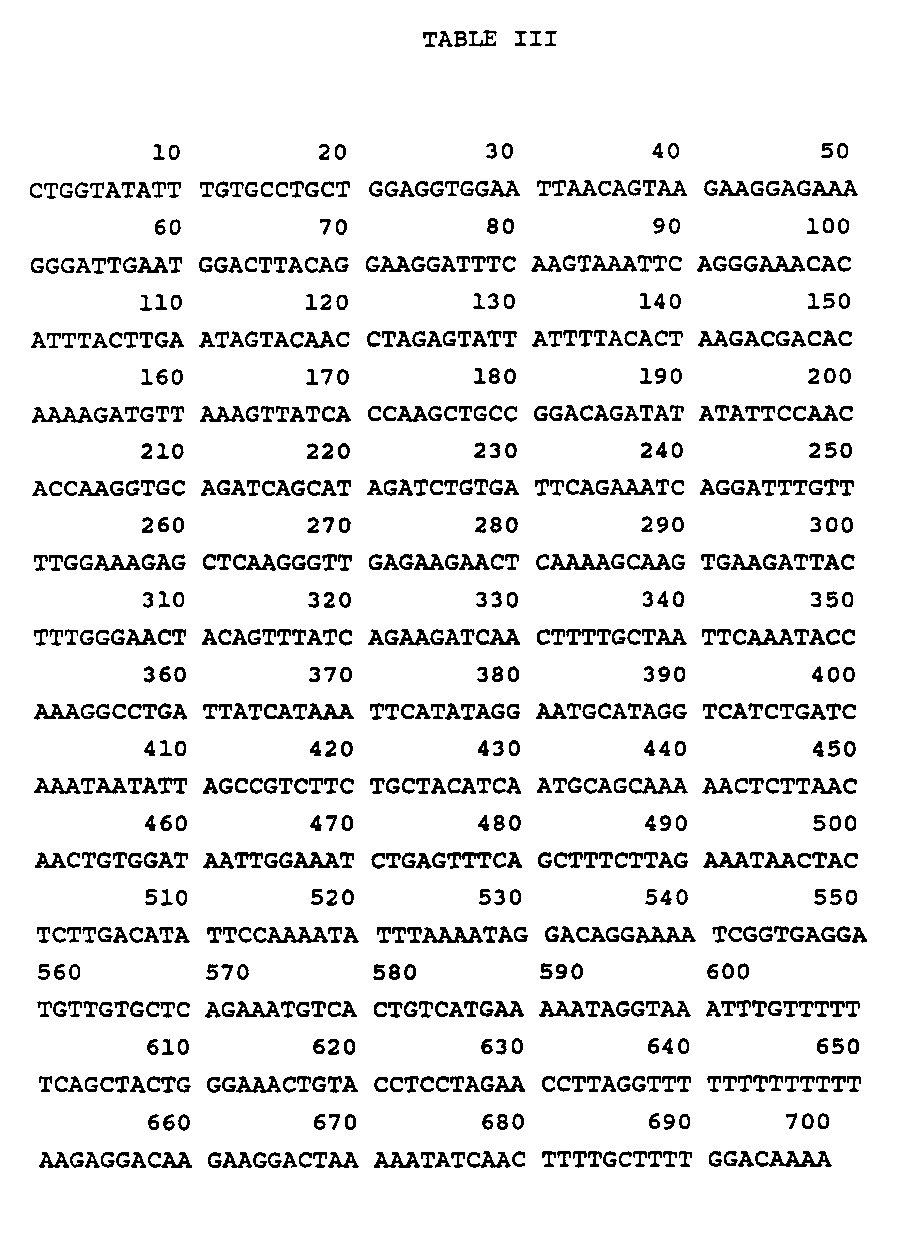

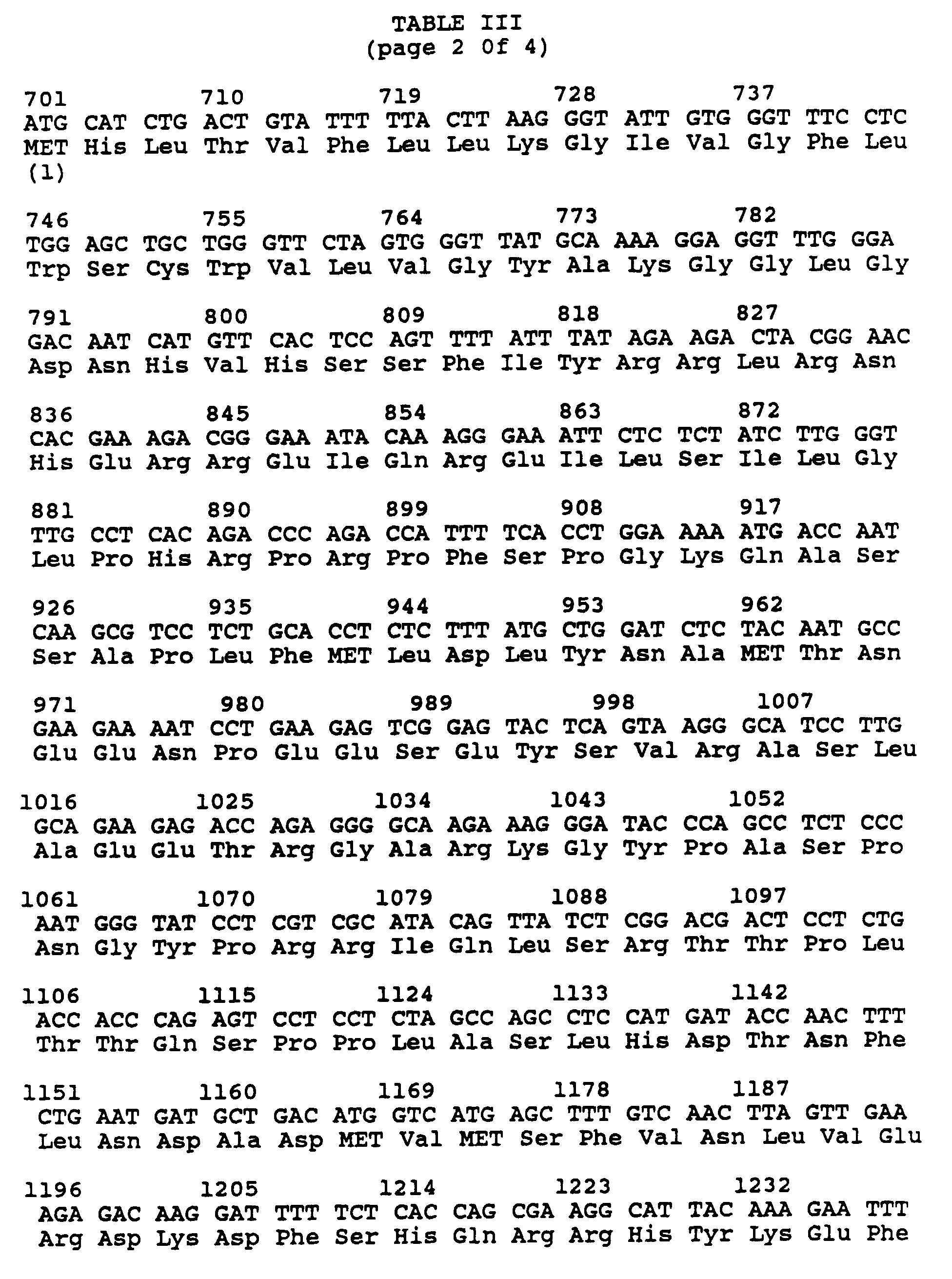

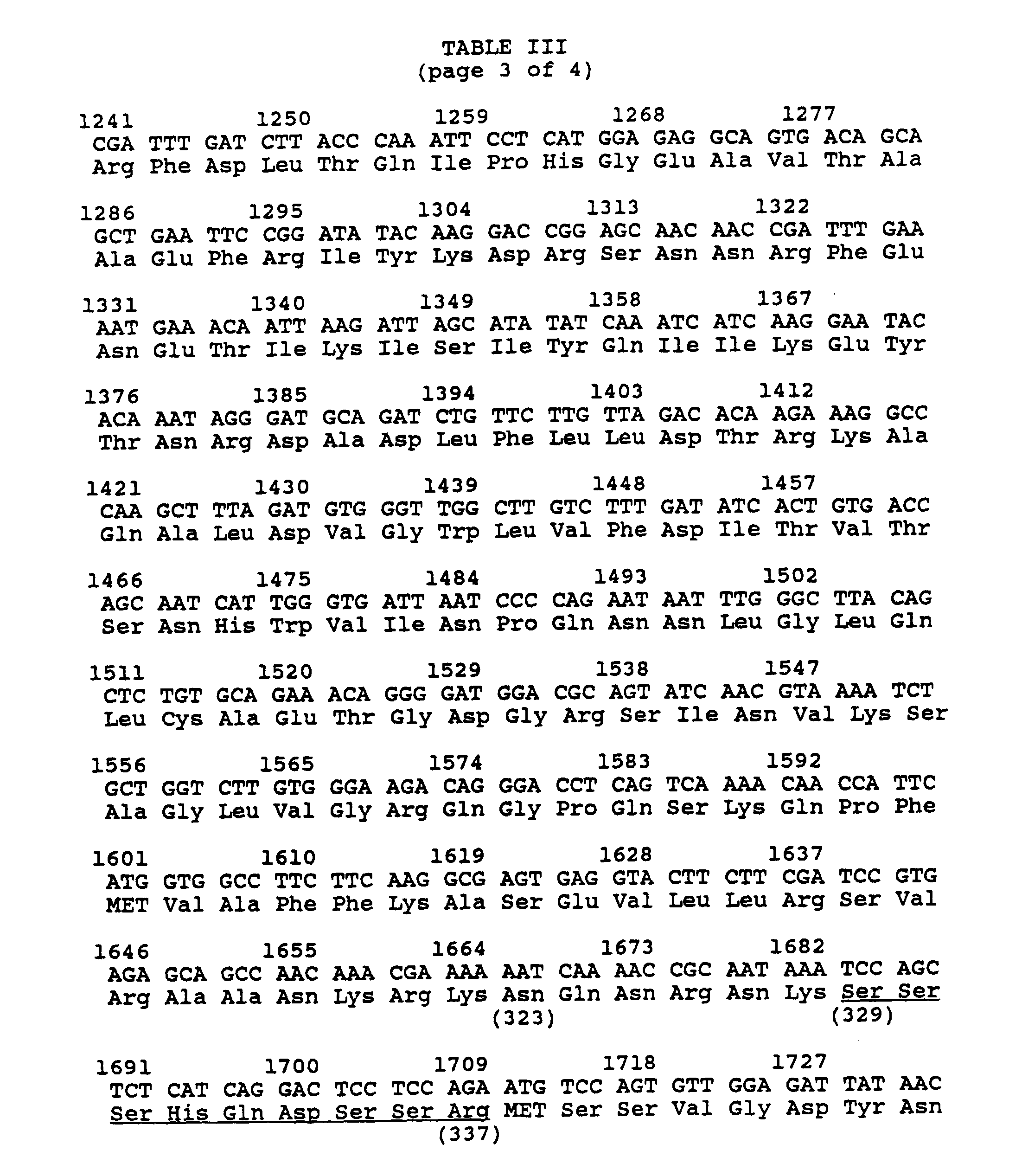

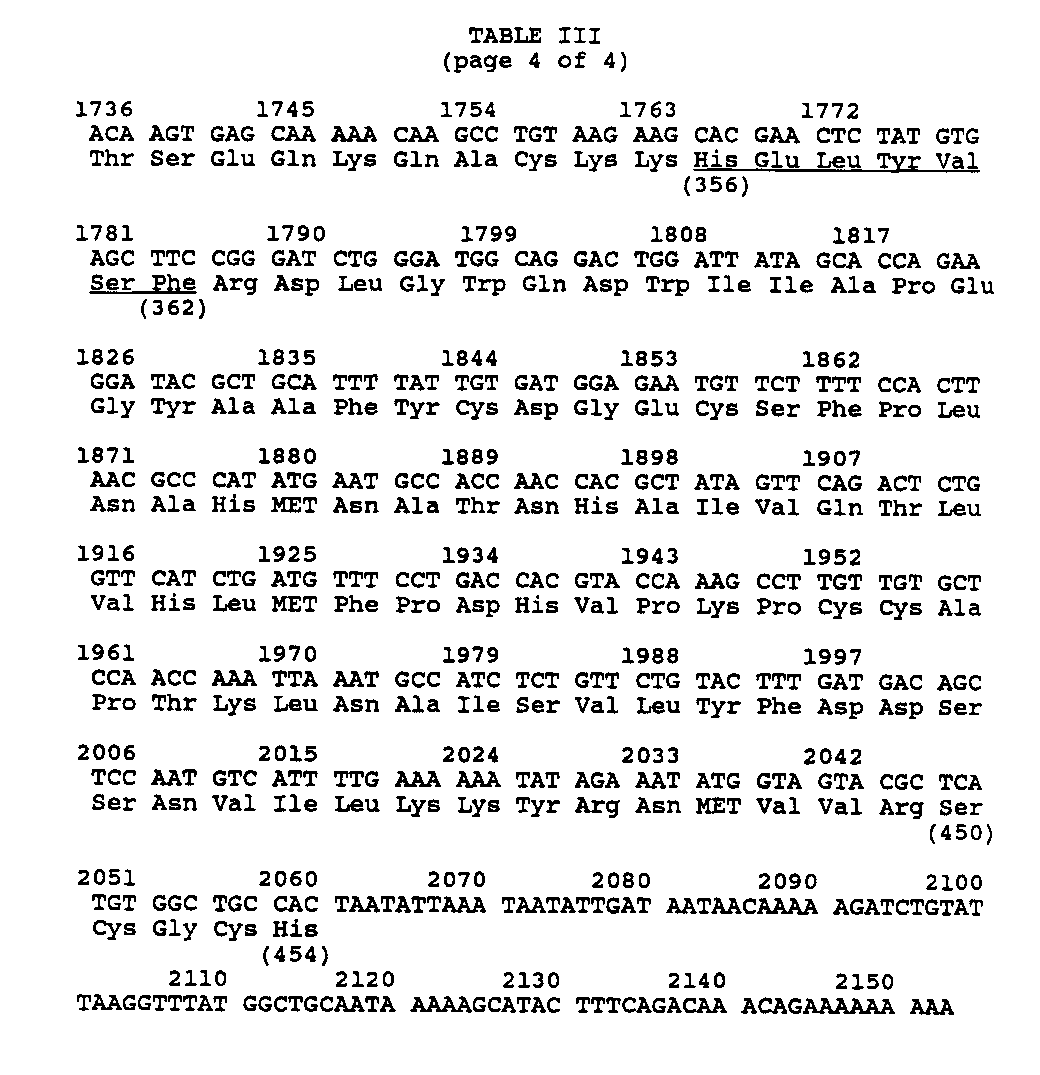

The tryptic sequence His-Glu-Leu-Tyr-Val-Ser-Phe-(Ser) described above is noted to be similar to the sequence His-Pro-Leu-Tyr-Val-Asp-Phe-Ser found in the bovine and human cartilage/bone protein BMP-2A sequence, for instance as described in Publication WO 88/00205. Human BMP-5 shares homology with other BMP molecules as well as other members of the TGF-β superfamily of molecules. The cysteine-rich carboxy-terminal 102 amino acid residues of human BMP-5 shares the following homologies with BMP proteins disclosed herein and in Publications WO 88/00205 and WO 89/10409 described above: 61% identity with BMP-2; 43% identity with BMP-3, 59% identity with BMP-4; 91% identity with BMP-6; and 88% identity with BMP-7. Human BMP-5 further shares the following homologies: 38% identity with TGF-β3: 37% identity with TGF-β2; 36% identity with TGF-β1: 25% identity with Mullerian Inhibiting Substance (MIS), a testicular glycoprotein that causes regression of the Mullerian duct during development of the male embryo; 25% identity with inhibin a; 38% identity with inhibin β_{B}; 45% identity with inhibin β_{A}; 56% identity with Vgl, a Xenopus factor which may be involved in mesoderm induction in early embryogenesis (Weeks and Melton, Cell 51:861-867 (1987)]; and 57% identity with Dpp the product of the Drosophila decapentaplegic locus which is required for dorsal-ventral specification in early embryogenesis and is involved in various other developmental processes at later stages of development [Padgett, et al., Nature 325:81-84 (1987)].

### C. Human BMP-6 Proteins

Six clones which hybridize to the second probe described in Example V.A. more strongly than to the third are picked and transformed into plasmids. Restriction mapping, Southern blot analysis, and DNA sequence analysis of these plasmids indicate that there are two classes of clones. Clones U2-7 and U2-10 contain human BMP-6 coding sequence based on their stronger hybridization to the second probe and closer DNA homology to the bovine BMP-6 sequence of Table II than the other 4 clones. DNA sequence data derived from these clones indicates that they encode a partial polypeptide of 132 amino acids comprising the carboxy-terminus of the human BMP-6 protein. U2-7 was deposited with the American Type Culture Collection (ATCC), Rockville, Maryland on June 23, 1989 under accession number 68021 under the provisions of the Budapest Treaty.

A primer extended cDNA library is made from U-2 OS mRNA using the oligonucleotide GGAATCCAAGGCAGAATGTG, the sequence being based on the 3' untranslated sequence of the human BMP-6 derived from the clone U2-10. This library is screened with an oligonucleotide of the sequence CAGAGTCGTAATCGC, derived from the BMP-6 coding sequence of U2-7 and U2-10. Hybridization is in standard hybridization buffer (SHB) at 42 degrees centigrade, with wash conditions of 42 degrees centigrade, 5X SSC, 0.1% SDS. Positively hybridizing clones are isolated. The DNA insert of one of these clones, PEH6-2, indicates that it extends further in a 5' direction than either U2-7 or U2-10. A primer extended cDNA library constructed from U-20S mRNA as above is screened with an oligonucleotide of the sequence GCCTCTCCCCCTCCGACGCCCCGTCCTCGT, derived from the sequence near the 5' end of PEH6-2. Hybridization is at 65 degrees centigrade in SHB, with washing at 65 degrees centigrade in 2X SSC, 0.1% SDS. Positively hybridizing recombinants are isolated and analyzed by restriction mapping and DNA sequence analysis.

The 5' sequence of the insert of one of the positively hybridizing recombinants, PE5834#7, is used to design an oligonucleotide of the sequence CTGCTGCTCCTCCTGCTGCCGGAGCGC. A random primed cDNA library [synthesized as for an oligo (dT) primed library except that (dN)₆ is used as the primer] is screened with this oligonucleotide by hybridization at 65 degrees centigrade in SHB with washing at 65 degrees centigrade in 1X SSC, 0.1% SDS. A positively hybridizing clone, RP10, is identified, isolated, and the DNA sequence sequence from the 5' end of its insert is determined. This sequence is used to design an Oligonucletide of the sequence TCGGGCTTCCTGTACCGGCGGCTCAAGACGCAGGAGAAGCGGGAGATGCA. A human placenta cDNA library (Stratagene catalog #936203) is screened with this oligonucleotide by hybridization in SHB at 65 degrees centigrade, and washing at 65 degrees centigrade with 0.2 X SSC, 0.1% SDS. A positively hybridizing recombinant designated BMP6C35 is isolated. DNA sequence analysis of the insert of this recombinant indicates that it encodes the complete human BMP-6 protein. BMP6C35 was deposited with the American Type Culture Collection, 12301 Parklawn Drive, Rockville, Maryland USA on March 1, 1990 under Accession Number 68245 under the provisions of the Budapest Treaty.

The DNA and derived amino acid sequence of the majority of the insert of BMP6C35 is given in Table IV. This DNA sequence contains an open reading frame of 1539 base pairs which encodes the 513 amino acid human BMP-6 protein precursor. The presumed initiator methionine codon is preceded by a 5'untranslated sequence of 159 base pairs with stop codons in all three reading frames. The stop codon at nucleotides 1699-1701 is followed by at least 1222 base pairs of 3'untranslated sequence. It is noted that U2-7 has a C residue at the position corresponding to the T residue at position 1221 of BMP6C35; U2-7 also has a C residue at the position corresponding to the G residue at position 1253 of BMP6C35. These do not cause amino acid differences in the encoded proteins, and presumably represent allelic variations.

The oligonucleotide TCGGGCTTCCTGTACCGGCGGCTCAAGACGCAGGAGAAGCGGGAGATGCA is used to screen a human genomic library (Toole et al supra), by hybridizing nitrocellulose replicas of 1 X 10⁶ recombinants with the oligonucleotide in SHB at 65 degrees centigrade, and washing at 65 degrees centigrade with 0.2 X SSC, 0.1% SDS. Positively hybridizing clones are purified. The oligonucleotide hybridizing region is localized to an approximately 1.5 kb Pst I fragment. DNA sequence analysis of this fragment confirms the 5' sequence indicated in Table IV.

The first underlined portion of the sequence in Table IV from amino acid #388 to #396, Ser-Thr-Gln-Ser-Gln-Asp-Val-Ala-Arg, corresponds to the similar sequence Ser-Thr-Pro-Alg-Gln-Asp-Val-Ser-Arg of the bovine sequence described above and set forth in Table II. The second underlined sequence in Table IV from amino acid #415 through #421 His-Glu-Leu-Tyr-Val-Ser-Phe, corresponds to the tryptic fragment identified above from which the oligonucleotide probes are designed. The tryptic sequence His-Glu-Leu-Tyr-Val-Ser-Phe-(Ser) is noted to be similar to a sequence found in other BMP proteins for example the sequence His-Pro-Leu-Tyr-Val-Asp-Phe-Ser found in the bovine and human cartilage/bone protein BMP-2 sequence as described in Publication WO 88/00205. BMP-6 therefore represents a new member of the BMP subfamily of TGF-β like molecules which includes the molecules BMP-2, BMP-3, BMP-4 described in Publications WO 88/00205 and WO 89/10409, as well as BMP-5 and BMP-7 described herein.

Based on knowledge of other BMP proteins, as well as other proteins in the TGF-β family, BMP-6 is predicted to be synthesized as a precursor molecule and the precursor polypeptide would be cleaved between amino acid #381 and amino acid #382 yielding a 132 amino acid mature polypeptide with a calculated molecular weight of approximately 15Kd. The mature form of BMP-6 contains three potential N-linked glycosylation sites per polypeptide chain as does BMP-5.

The processing of BMP-6 into the mature form is expected to involve dimerization and removal of the N-terminal region in a manner analogous to the processing of the related protein TGF-β [L.E. Gentry, et al., (1988); R. Dernyck, et al., (1985) supra]. It is contemplated that the active BMP-6 protein molecule is a dimer. It is further contemplated that the mature active species of BMP-5 comprises protein molecule is a homodimer comprised of two polypeptide subunits each subunit comprising amino acid #382 - #513 as set forth in Table IV. Further active species of BMP-6 are contemplated such as phoprotein dimers or a proprotein subunit and a mature subunit. Additional active BMP-5 proteins may comprise amino acid #388 - #513 thereby including the tryptic fragments found in the purified bovine material. Another BMP-5 protein of the invention comprises amino acid #412 - #513 thereby including the first conserved cystine residue.

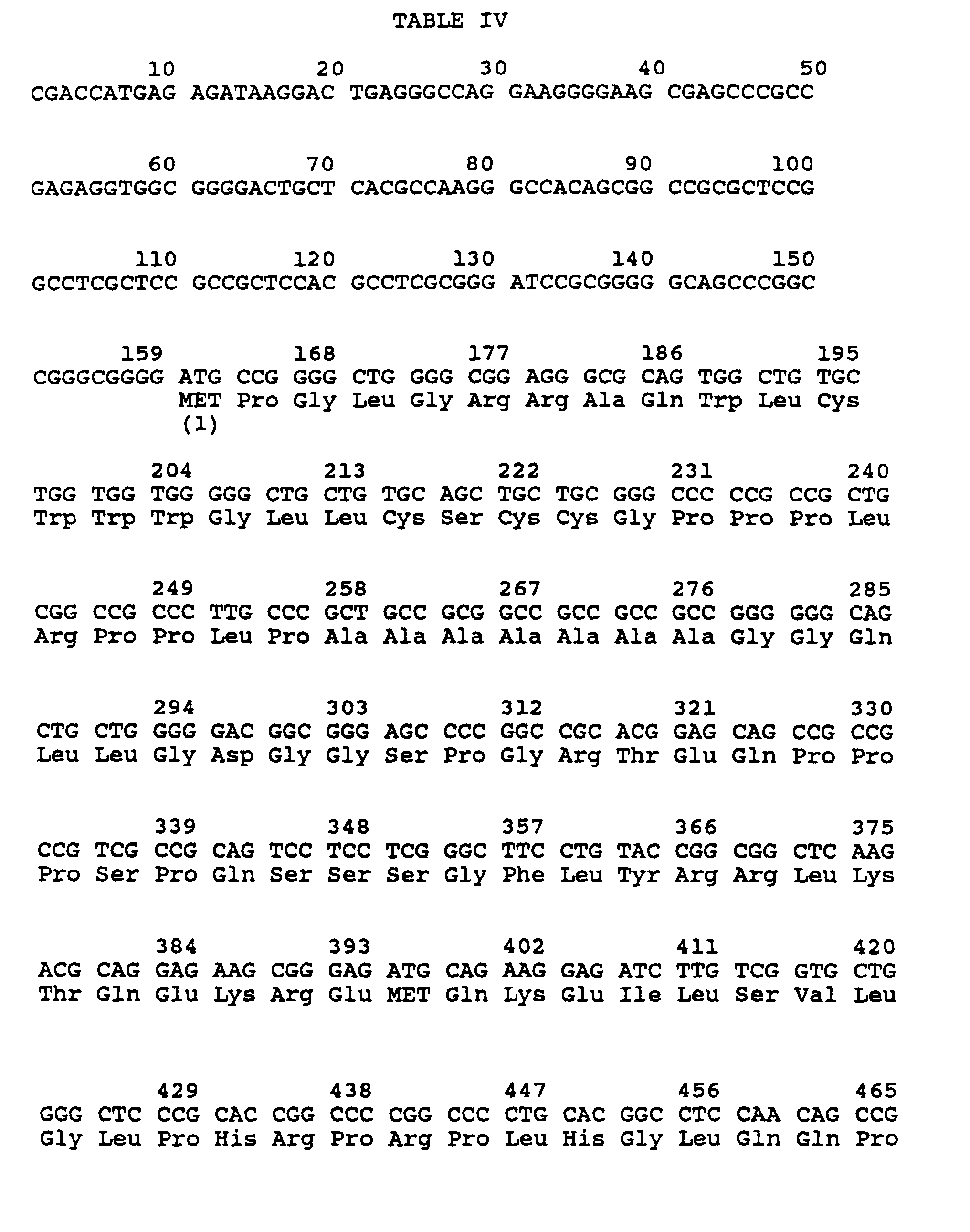

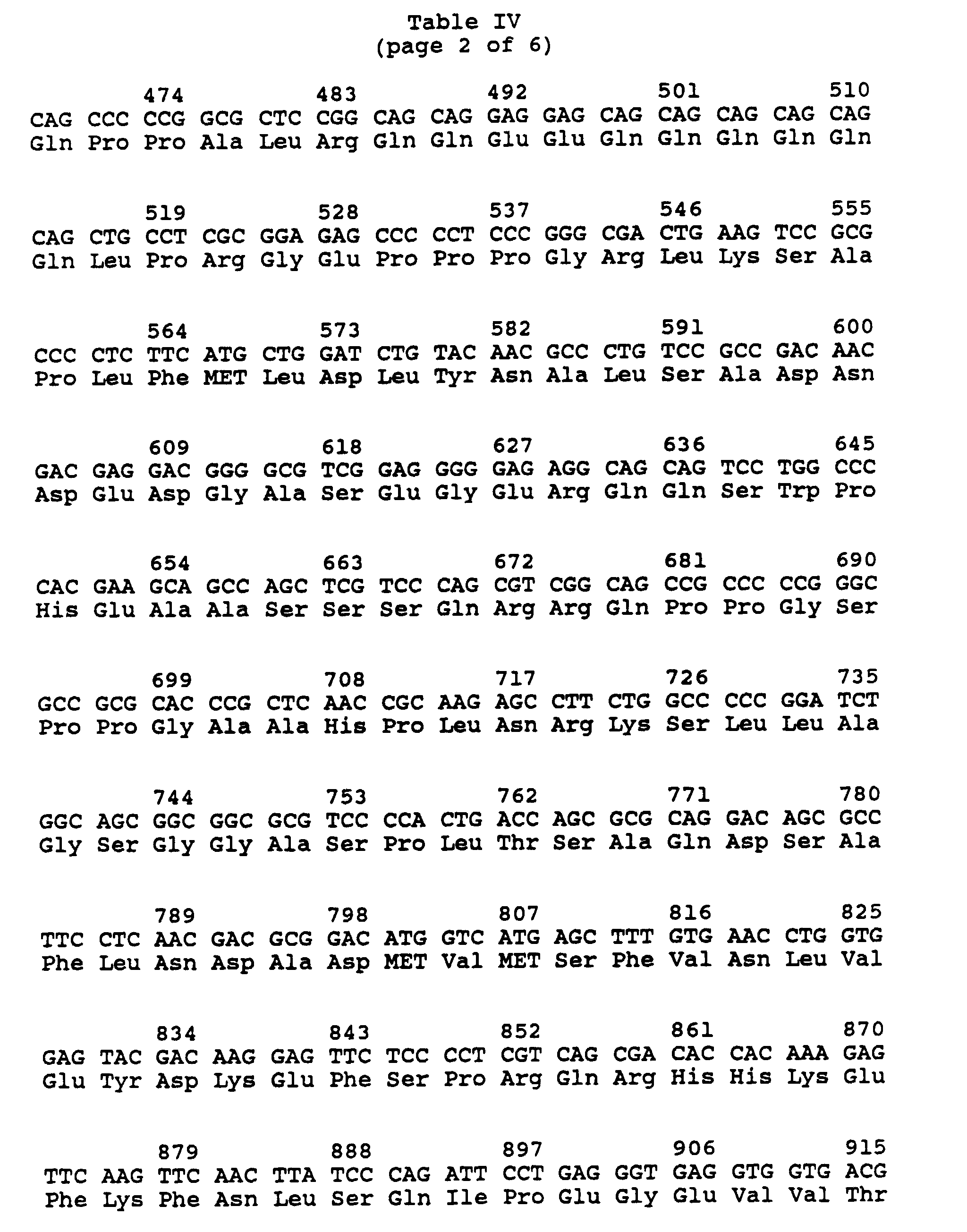

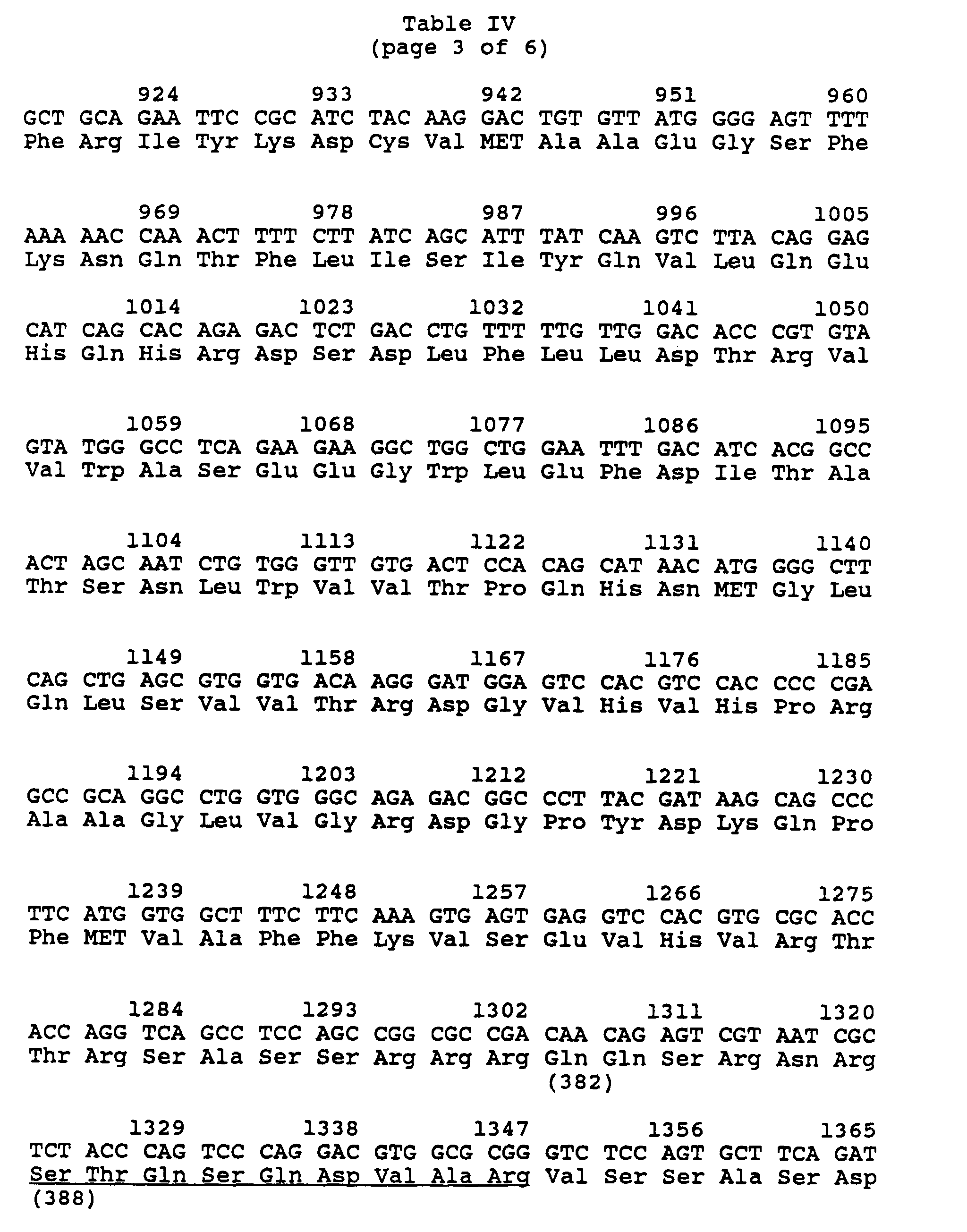

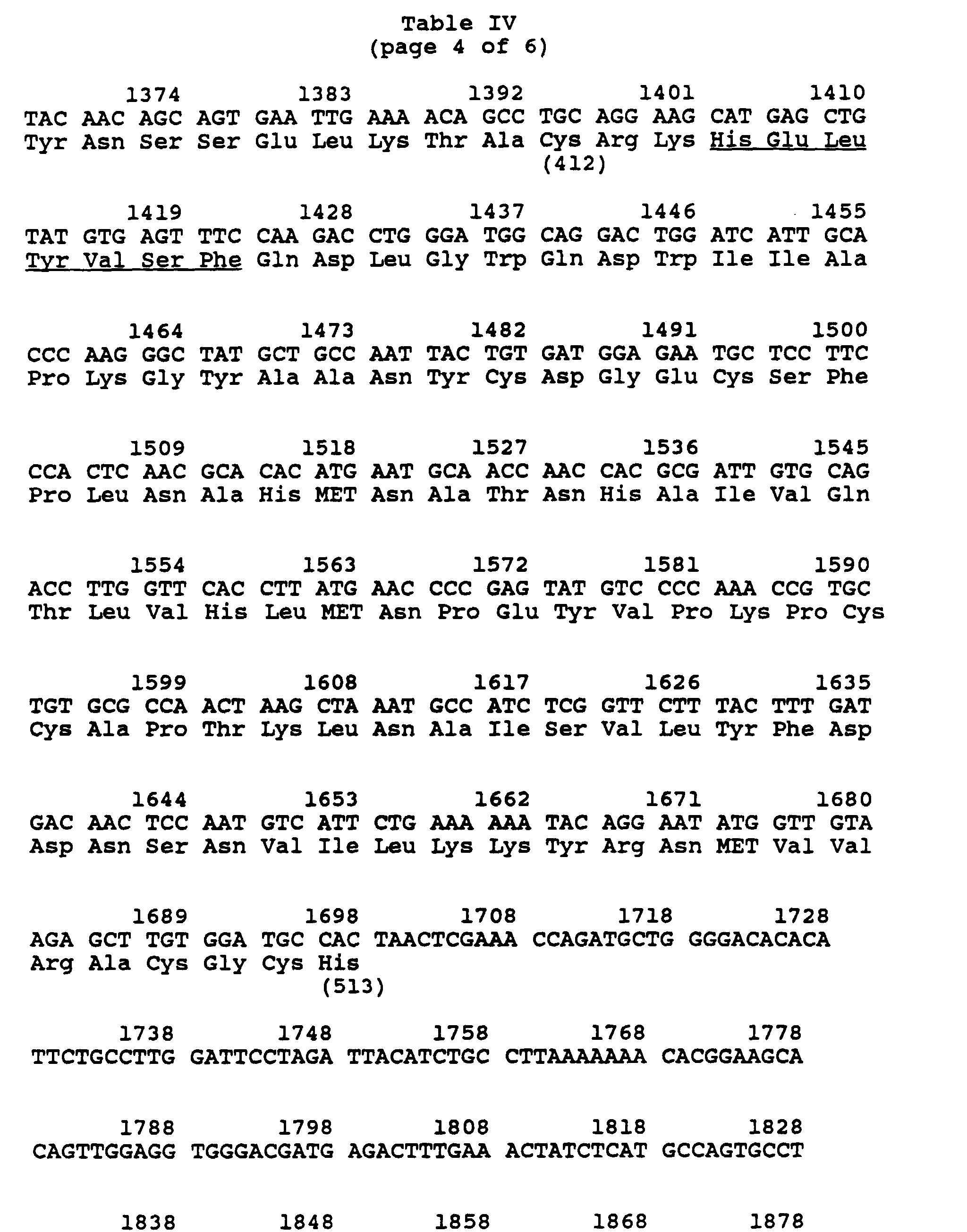

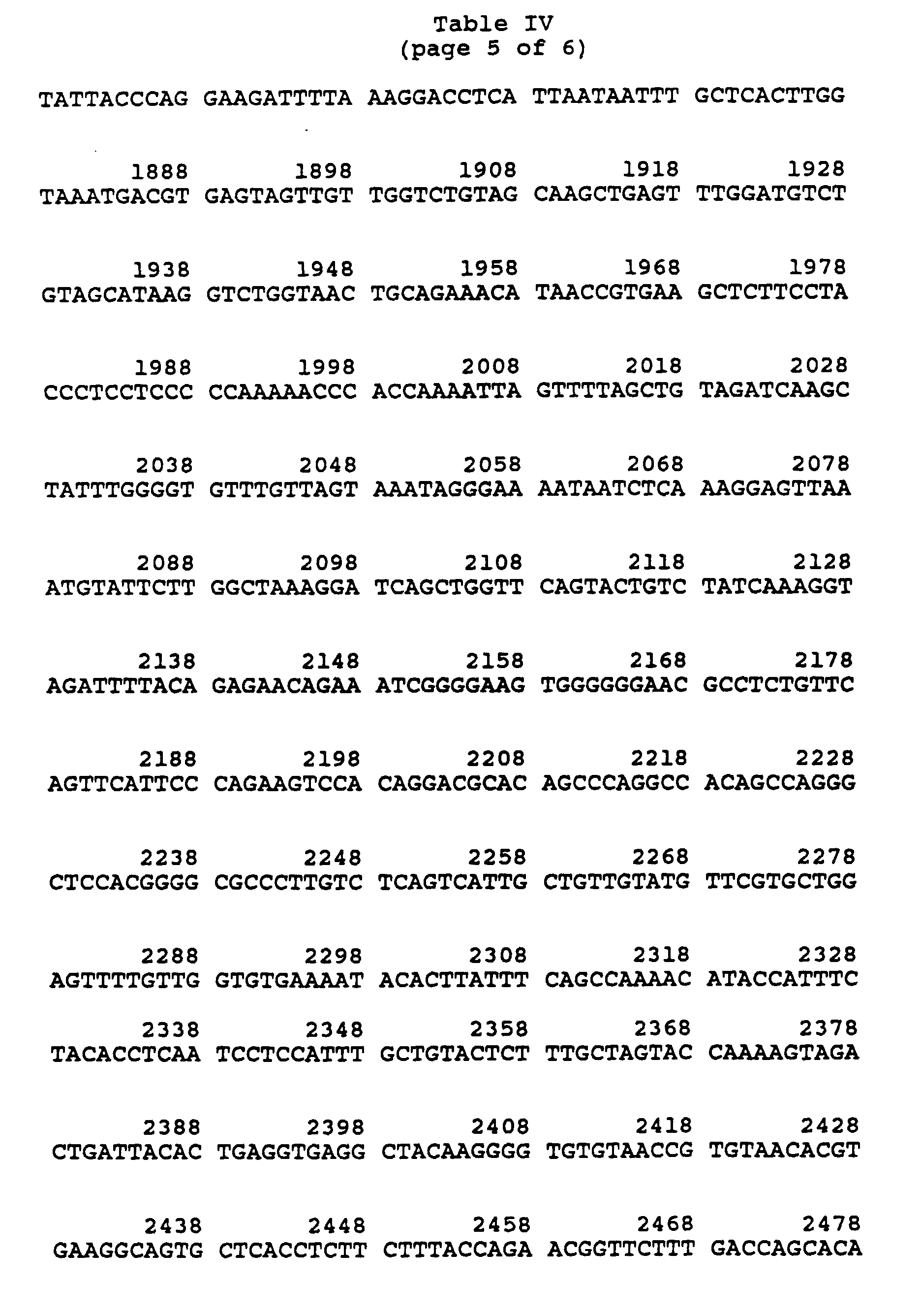

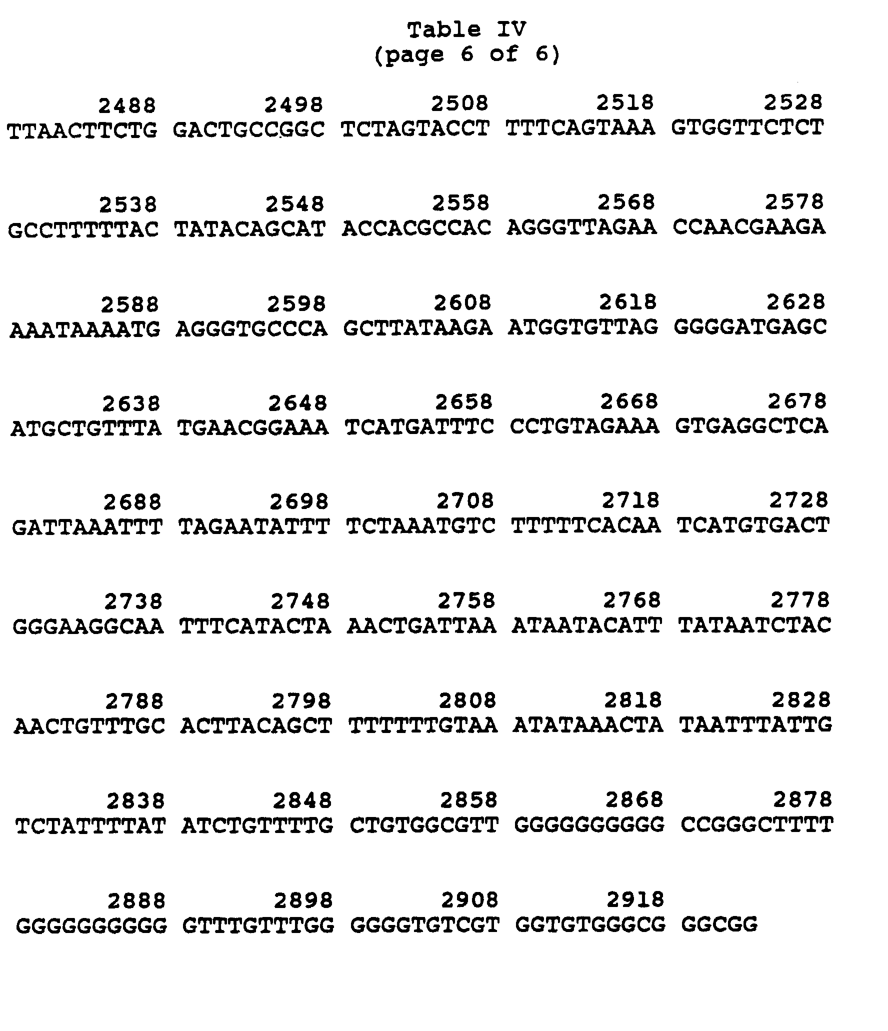

Comparision of the sequence of murine Vgr-1 [Lyons, et al., PNAS 86:4554 (1989)] to human BMP-6 reveals a degree of amino acid sequence identity greater than 92% The murine Vgr-1 is likely the murine homologue of BMP-6. Human BMP-6 shares homology with other BMP molecules as well as other members of the TGF-β superfamily of molecules. The cysteine-rich carboxy-terminal 102 amino acid residues of human BMP-6 shares the following homologies with BMP proteins disclosed herein and in Publications WO 88/00205 and WO 89/10409: 61% identity with BMP-2; 44% identity with BMP-3, 60% identity with BMP-4; 91% identity with BMP-5; and 87% identity with BMP-7. Human BMP-6 further shares the following homologies: 41% identity with TGF-β3; 39% identity with TGF-β2; 37% identity with TGF-β1; 26% identity with Mullerian Inhibiting Substance (MIS), a testicular glycoprotein that causes regression of the Mullerian duct during development of the male embryo; 25% identity with inhibin α ; 43% identity with inhibin β_{B}; 49% identity with inhibin β_{A}; 58% identity with Vgl, a Xenopus factor which may be involved in mesoderm induction in early embryogenesis (Weeks and Melton, (1987) Supra]; and 59% identity with Dpp the product of the Drosophila decapentaplegic locus which is required for dorsal-ventral specification in early embryogenesis and is involved in various other developmental processes at later stages of development [Padgett, et al., (1987) supra].

### D. Human BMP-7 Proteins

The other four clones of Example V.C. above which appear to represent a second class of clones encode a novel polypeptide which we designate as BMP-7. One of these clones, U2-5, was deposited with the ATCC on June 22, 1989 under accession number ATCC 68020 under the provisions of the Budapest Treaty. This clone was determined not to contain the entire coding sequence for BMP-7. An oligo of the squence GCGAGCAATGGAGGATCCAG (designed on the basis of the 3' noncoding sequence of U2-5) was used to make a primer-extended cDNA library from U-2 OS mRNA (Toole, et al.). 500,000 recombinants of this library were screened with the oligonucleotide GATCTCGCGCTGCAT (designed on the basis of the BMP-7 coding sequence) by hybridization in SHB at 42° and washing in 5X SSC, 0.1% SDS at 42°. Several hybridizing clones were obtained. DNA sequence analysis and derived amino acid sequence of one of these clones, PEH7-9, is given in Table V. PEH7-9 was deposited with the American Type Culture Collection (ATCC), Rockville, Maryland on November 17, 1989 under accession number ATCC 68182 under the provisions of the Budapest Treaty. PEH7-9 contains an insert of 1448 base pairs. This clone, PEH7-9, is expected to contain all of the nucleotide sequence necessary to encode BMP-7 proteins. The cDNA sequence of Table V contains an open reading frame of 1292 base pairs, encoding a protein of 431 amino acids, preceded by a 5' untranslated region of 96 base pairs with stop codons in all frames, and contains a 3' untranslated region of 60 base pairs following the in frame stop codon TAG.

This protein of 431 amino acids has a molecular weight of 49,000 daltons as predicted by its amino acid sequence and is contemplated to represent the primary translation product. Based on knowledge of other BMP proteins as well as other proteins within the TGF-β family, it is predicted that the precursor polypeptide would be cleaved between amino acid #299 and #300, yielding a 132 amino acid mature peptide.

It is contemplated that processing of BMP-7 to the mature form involves dimerization of the proprotein and removal of the N-terminal region in a manner analogous to the processing of the related protein TGF-B [L.E. Gentry, et al., (1988) Supra and; R. Dernyck, et al., (1985) supra]. It is comtemplated therefore that the mature active species of BMP-7 comprises a homodimer of 2 polypeptide subunits each subunit cmprising amino acid #300 - #431 as shown in Table V with a calculated weight of 15,000 daltons. Other active BMP-7 species are contemplated, for example, protein dimers or proprotein subunits linked to mature subunits. Additional active species may comprise amino acids #309 - #431 of Table V such species including the tryptic sequences found in the purified bovine material. Also contemplated are BMP-7 proteins comprising amino acids #330-#431 thereby including the first conserved cysteine residue.

The underlined sequence of Table V from amino acid #309 - #314 Asn-Gln-Glu-Ala-Leu-Arg is the same sequence as that of tryptic fragment #5 found in the 28,000 - 30,000 dalton purified bone preparation as described in the "BMP" Publications WO 88/00205 and WO 89/10409 mentioned above. The underlined sequence of Table V from amino acid #333-#339 His-Glu-Leu-Tyr-Val-Ser-Phe corresponds to the tryptic fragment identified in the bovine bone preparation described above from which the oligonucleotide probes are designed.

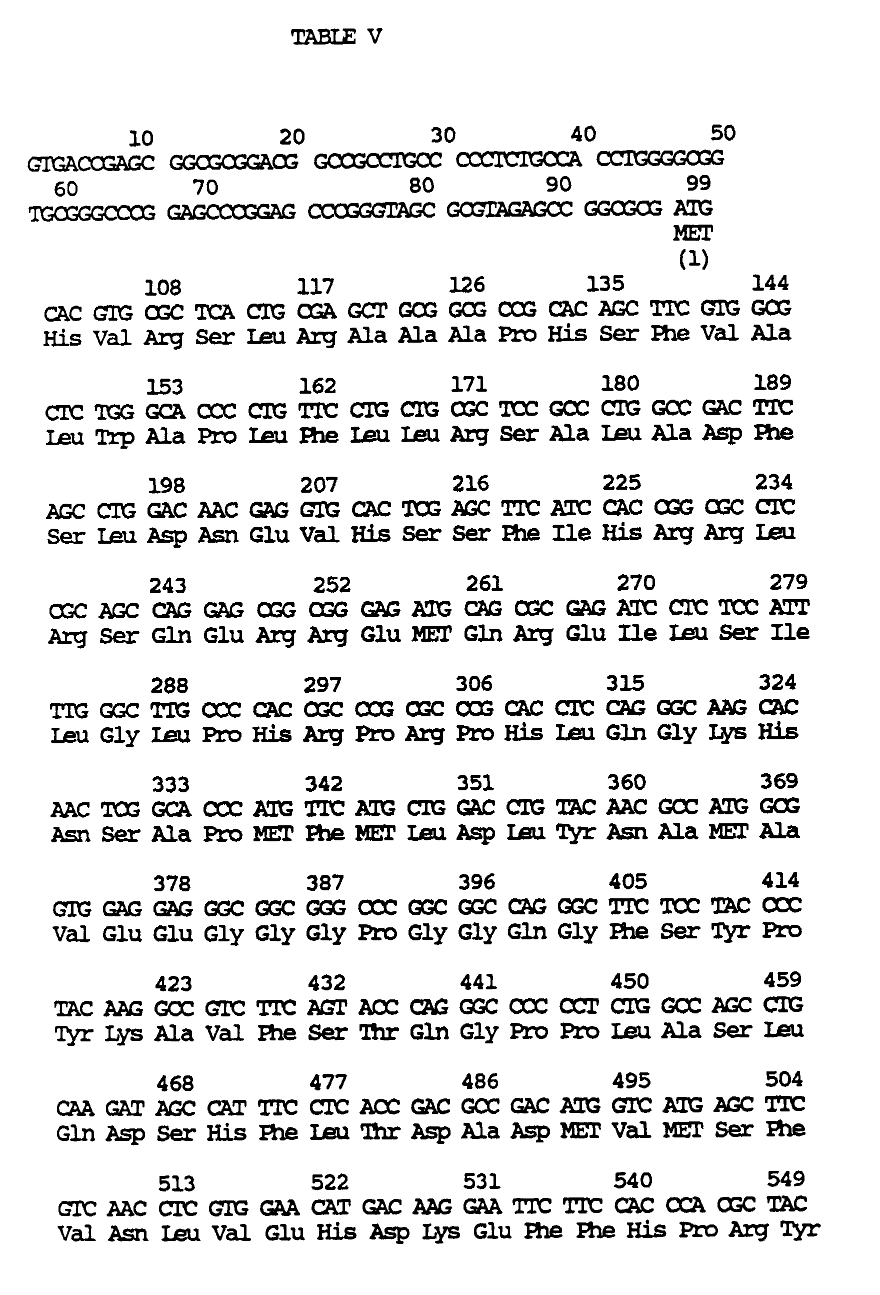

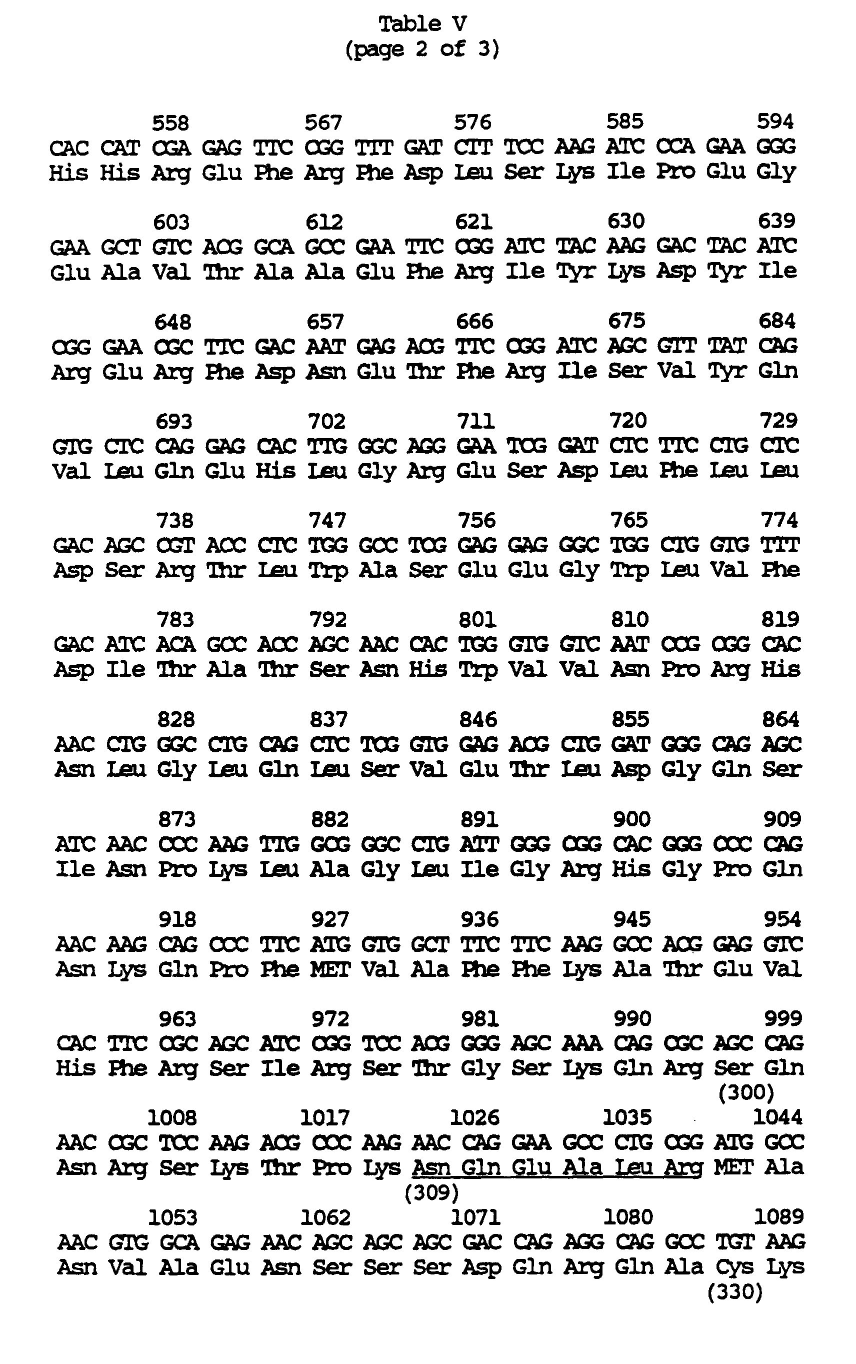

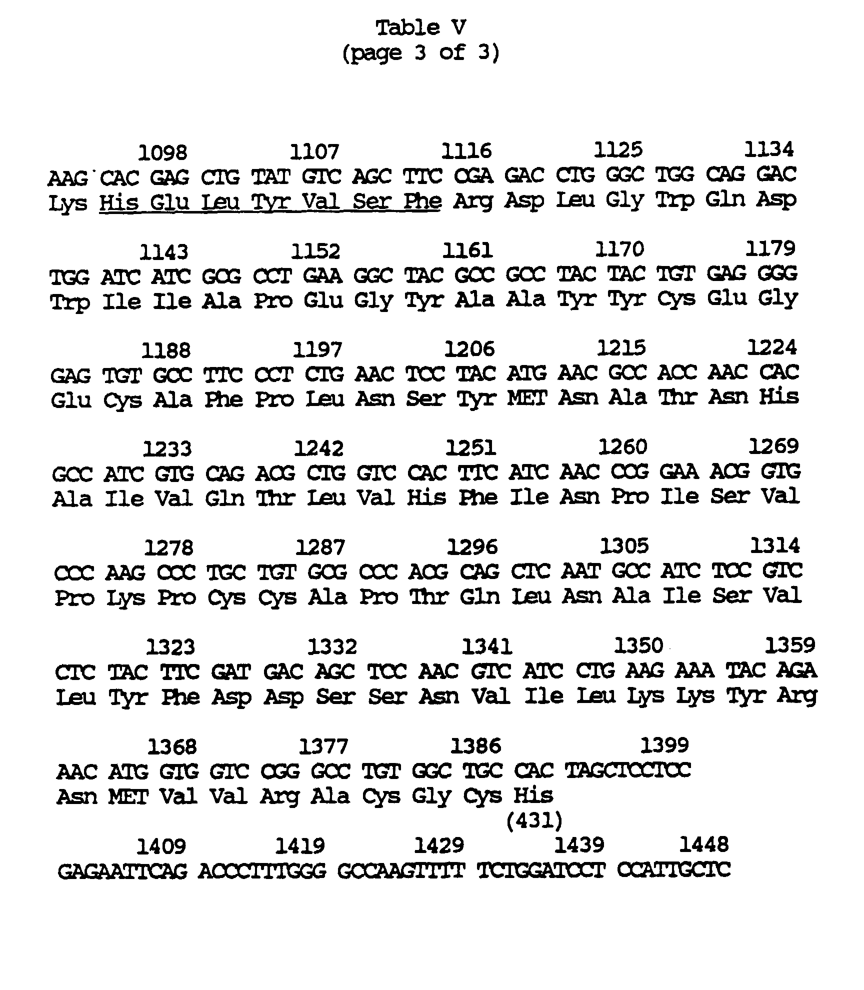

Like BMP-5 and BMP-6, human BMP-7 shares homology with other BMP molecules as well as other members of the TGF-β superfamily of molecules. The cysteine-rich carboxy-terminal 102 amino acids residues of human BMP-7 shares the following homologies with BMP proteins herein and in Publications WO 88/00205 and WO 89/10409 described above: 60% identity with BMP-2; 43% identity with BMP-3, 58% identity with BMP-4, 87% identity with BMP-6; and 88% identity with BMP-5. Human BMP-7 further shares the following homologies: 40% identity with TGF-β3; 40% identity with TGF-β2; 36% identity with TGF-β1; 29% identity with Mullerian Inhibiting Substance (MIS), a testicular glycoprotein that causes regression of the Mullerian duct during development of the male embryo; 25% identity with inhibin-α; 44% identity with inhibin-β_{B}; 45% identity with inhibin-β_{A}; 57% identity with Vgl, a Xenopus factor which may be involved in mesoderm induction in early embryogenesis [Weeks adn Melton, (1987) Supra.] ; and 58% identity with Dpp the product of the Drosophila decapentaplegic locus which is required for dorsal-ventral specification in early embryogenesis and is involved in various other developmental processes at later stages of development [Padgett, et al., (1987) Supra.].

The invention encompasses the genomic sequences of BMP-5, BMP-6 and BMP-7. To obtain these sequences the cDNA sequences described herein are utilized as probes to screen genomic libraries using techniques known to those skilled in the art.

The procedures described above and additional methods known to those skilled in the art may be employed to isolate other related proteins of interest by utilizing the bovine or human proteins as a probe source. Such other proteins may find similar utility in, inter alia, fracture repair, wound healing and tissue repair.

### EXAMPLE VI

### Expression of BMP Proteins

In order to produce bovine, human or other mammalian BMP-5, BMP-6 or BMP-7 proteins of the invention, the DNA encoding it is transfected into an appropriate expression vector and introduced into mammalian cells or other preferred eukaryotic or prokaryotic hosts by conventional genetic engineering techniques. It is contemplated that the preferred expression system for biologically active recombinant human proteins of the invention will be stably transformed mammalian cells. For transient expression, the cell line of choice is SV40 transformed African green monkey kidney COS-1 or COS-7 which typically produce moderate amounts of the protein encoded within the plasmid for a period of 1-4 days. For stable high level expression of BMP-5, BMP-6 or BMP-7 the preferred cell line is Cinese hamster Ovary (CHO). It is therefore contemplated that the preferred mammalian cells will be CHO cells.

The transformed host cells are cultured and the BMP proteins of the invention expressed thereby are recovered, isolated and purified. Characterization of expressed proteins is carried out using standard techiques. For example, characterization may include pulse labeling with [³5^{S}] methionine or cysteine and analysis by polyacrylamide electrophoresis. The recombinantly expressed BMP proteins are free of proteinaceous materials with which they are co-produced and with which they ordinarily are associated in nature, as well as from other contaminants, such as materials found in the culture media.

### A. Vector Construction

As described above, numerous expression vectors known in the art may be utilized in the expression of BMP proteins of the invention. The vector utilized in the following examples is pMT21, a derivitive of pMT₂, though other vectors may be suitable in practice of the invention.

pMT₂ is derived from pMT2-VWF, which has been deposited with the American Type Culture Collection (ATCC), Rockville, MD (USA) under accession number ATCC 67122 under the provisions of the Budapest Treaty. EcoRI digestion excises the cDNA insert present in pMT-VWF, yielding pMT2 in linear form which can be ligated and used to transform E. Coli HB 101 or DH-5 to ampicillin resistance. Plasmid pMT2 DNA can be prepared by conventional methods.

pMT21 is then constructed using loopout/in mutagenesis [Morinaga, et al., Biotechnology 84:636 (1984)]. This removes bases 1075 to 1170 (inclusive). In addition it inserts the following sequence: 5' TCGA 3'. This sequence completes a new restriction site, XhoI. This plasmid now contains 3 unique cloning sites PstI, EcoRI, and XhoI.

In addition, pMT21 is digested with EcoRV and XhoI, treating the digested DNA with Klenow fragment of DNA polymerase I and ligating ClaI linkers (NEBio Labs, CATCGATG). This removes bases 2171 to 2420 starting from the HindIII site near the SV40 origin of replication and enhancer sequences of pMT2 and introduces a unique Cla I site, but leaves the adenovirus VAI gene intact.

### B. BMP-5 Vector Construction

A derivative of the BMP-5 cDNA sequence set forth in Table III comprising the the nucleotide sequence from nucleotide #699 to #2070 is specifically amplified. The oligonucleotides CGACCTGCAGCCACCATGCATCTGACTGTA and TGCCTGCAGTTTAATATTAGTGGCAGC are utilized as primers to allow the amplification of nucleotide sequence #699 to #2070 of Table III from the insert of clone U2-16 described above in Example V. This procedure introduces the nucleotide sequence CGACCTGCAGCCACC immediately preceeding nucleotide #699 and the nucleotide sequence CTGCAGGCA immediately following nucleotide #2070. The addition of these sequences results in the creation of PstI restriction endonuclease recognition sites at both ends of the amplified DNA fragment. The resulting amplified DNA product of this procedure is digested with the restriction endonuclease PstI and subcloned into the PstI site of the pMT2 derivative pMT21 described above. The resulting clone is designated H5/5/pMT.

The insert of H5/5/pMT is excised by PstI digestion and subcloned into the plasmid vector pSP65 at the PstI site resulting in BMP5/SP6. BMP5/SP6 and U2-16 are digested with the restriction endonucleases NsiI and NdeI to excise the portion of their inserts corresponding to nucleotides #704 to #1876 of Table III. The resulting 1173 nucleotide NsiI-Ndei fragment of clone U2-16 is ligated into the NsiI-NdeI site of BMP5/SP6 from which the corresponding 1173 nucleotide NsiI-NdeI fragment had been removed. The resulting clone is designated BMP5mix/SP64.

Direct DNA sequence analysis of BMP5mix/SP64 is performed to confirm identity of the nucleotide sequences produced by the amplification to those set forth in Table III. The clone BMP5mix/SP64 is digested with the restriction endonuclease PstI resulting in the excision of an insert comprising the nucleotides #699 to #2070 of Table III and the additional sequences containing the PstI recognition sites as described above. The resulting 1382 nucleotide PstI fragment is subcloned into the PstI site of the pMT2 derivative pMT21. This clone is designated BMP5mix/pMT21#2.

### C. BMP-6 Vector Construction

A derivative of the BMP-6 cDNA sequence set forth in Table IV comprising the nucleotide sequence from nucleotide #160 to #1706 is produced by a series of techniques known to those skilled in the art. The clone BMP6C35 described above in Example V is digested with the restriction endonucleases ApaI and TaqI, resulting in the excision of a 1476 nucleotide portion of the insert comprising nucleotide #231 to #1703 of the sequence set forth in Table IV. Synthetic olignucloetides with SalI restriction endonuclease site converters are designed to replace those nucleotides corresponding to #160 to #230 and #1704 to #1706 which are not contained in the 1476 ApaI-TaqI fragment of the BMP-6 cDNA sequence. Oligonucleotide/SalI converters conceived to replace the missing 5' (TCGACCCACCATGCCGGGGCTGGGGCGGAGGGCGCAGTGGCTGTG CTGGTGGT GGGGGCTGTGCTGCAGCTGCTGCGGGCC and CGCAGCAGCTGCACAGCAGCCCCCACCACCAGCACAGCCACTGCGCC CTCCGCCCCAG CCCCGGCATGGTGGG)and 3' (TCGACTGGTTT and CGAAACCAG) sequences are annealed to each other independently. The annealed 5' and 3' converters are then ligated to the 1476 nucleotide ApaI-TaqI described above, creating a 1563 nucleotide fragment comprising the nucleotide sequence from #160 to #1706 of Table IV and the additional sequences contrived to create SalI restriction endonuclease sites at both ends. The resulting 1563 nucleotide fragment is subcloned into the SalI site of pSP64. This clone is designated BMP6/SP64#15.

DNA sequence analysis of BMP6/SP64#15 is performed to confirm identity of the 5' and 3' sequences replaced by the converters to the sequence set forth in Table IV. The insert of BMP6/SP64#15 is excised by digestion with the restriction endonuclease SalI. The resulting 1563 nucleotide SalI fragment is subcloned into the XhoI restriction endonuclease site of the pMT2 derivative pMT21 and designated herein as BMP6/pMT21.

### D. BMP-7 Vector Construction

A derivative of the BMP-7 sequence set forth in Table V comprising the nucleotide sequence from nucleotide #97 to #1402 is specifically amplified. The oligonucleotides CAGGTCGACCCACCATGCACGTGCGCTCA and TCTGTCGACCTCGGAGGAGCTAGTGGC are utilized as primers to allow the amplification of nucleotide sequence #97 to #1402 of Table V from the insert of clone PEH7-9 described above. This procedure generates the insertion of the nucleotide sequence CAGGTCGACCCACC immediately preceeding nucleotide #97 and the insertion of the nucleotide sequence GTCGACAGA immediately following nucleotide #1402. The addition of these sequences results in the creation of a SalI restriction endonuclease recognition site at each end of the amplified DNA fragment. The resulting amplified DNA product of this procedure is digested with the restriction endonuclease SalI and subcloned into the SalI site of the plasmid vector pSP64 resulting in BMP7/SP6#2.

The clones BMP7/SP6#2 and PEH7-9 are digested with the restriction endonucleases NcoI And StuI to excise the portion of their inserts corresponding to nucleotides #363 to #1081 of Table V. The resulting 719 nucleotide NcoI-StuI fragment of clone PEH7-9 is ligated into the NcoI-StuI site of BMP7/SP6#2 from which the corresponding 719 nucleotide fragment is removed. The resulting clone is designated BMP7mix/SP6.

Direct DNA sequence analysis of BMP7mix/SP6 confirmed identity of the 3' region to the nucleotide sequence from #1082 to #1402 of Table V, however the 5' region contained one nucleotide misincorporation.

Amplification of the nucleotide sequence (#97 to #1402 of Table V) utilizing PEH7-9 as a template is repeated as described above. The resulting amplified DNA product of this procedure is digested with the restriction endonucleases SalI and PstI. This digestion results in the excision of a 747 nucleotide fragment comprising nucleotide #97 to #833 of Table V plus the additional sequences of the 5' priming oligonucleotide used to create the SalI restriction endonuclease recognition site described earlier. This 747 SalI-PstI fragment is subcloned into a SalI-PstI digested pSP65 vector resulting in 5'BMP7/SP65. DNA sequence analysis demonstrates that the insert of the 5'BMP7/SP65#1 comprises a sequence identical to nucleotide #97 to #362 of Table V.

The clones BMP7mix/SP6 and 5'BMP7/SP65 are digested with the restriction endonucleases SalI and NcoI. The resulting 3' NcoI-SalI fragment of BMP7mix/SP6 comprising nucleotides #363 to #1402 of Table V and 5' SalI-NcoI fragment of 5'BMP7/SP65 comprising nucleotides #97 to #362 of Table V are ligated together at the NcoI restriction sites to produce a 1317 nucleotide fragment comprising nucleotides #97 to #1402 of Table V plus the additional sequences derived from the 5' and 3' oligonucleotide primers which allows the creation of SalI restriction sites at both ends of this fragment. This 1317 nucleotide SalI fragment is ligated into the SalI site of the pMT2 derivative pMT2Cla-2. This clone is designated BMP7/pMT2.

The insert of BMP7/pMT2 is excised by digestion with the restriction endonuclease SalI. The resulting 1317 nucleotide SalI fragment is subcloned into the SalI restriction site of the vector pSP64. This clone is designated BMP7/SP64#2d. The insert of BMP7/SP64#2d is excised by digestion with SalI and the resulting SalI fragment comprising nucleotides #97 to #1402 of Table V is subcloned into the XhoI restriction endonuclease site of the pMT2 derivative pMT21 described above.

### Example VII

### Transient COS Cell Expression

To obtain transient expression of BMP-5, BMP-6, and BMP-7 proteins one of the vectors containing the cDNA for BMP-5, BMP-6 or BMP-7, BMP5mix/pMT21#2, BMP6/pMT21#2, or BMP7/pMT21 respectively, are transfected into COS-1 cells using the electroporation method. Other suitable transfection methods include DEAE-dextran, and lipofection. Approximately 48 hours later, cells are analysed for expression of both intracellular and secreted BMP-5, BMP-6 or BMP-7 protein by metabolic labelling with [³⁵S] methionine and polyacrylamide gel electrophoresis. Intracellular BMP is analyzed in cells which are treated with tunicamycin, an inhibitor of N-linked glycosylation. In tunicamycin-treated cells, the nonglycosylated primary translation product migrates as a homogeneous band of predictable size and is often easier to discern in polyacrylamide gels than the glycosylated form of the protein. In each case, intracelluar protein in tunicamycin-treated cells is compared to a duplicate plate of transfected, but untreated COS-1 cells.

### A. BMP-5 COS Expression

The results demonstrate that intracellular forms of BMP-5 of approximately 52 Kd and 57 Kd are made by COS cells. The 52 Kd protein is the size predicted by the primary sequence of the the BMP-5 cDNA clone. Following treatment of the cells with tunicamycin, only the 52 Kd form of BMP-5 is made, suggesting that the 57 Kd protein is a glycosylated derivative of the 52 Kd primary translation product. The 57 Kd protein is secreted into the conditioned medium and is apparently not efficiently processed by COS-1 cells into the pro and mature peptides.

### B. BMP-6 COS Expression

Intracellular BMP-6 exists as a doublet of approximately 61 Kd and 65 Kd in untreated COS-1 cells. In the presence of tunicamycin, only the 61 Kd protein is observed, indicating that the 65 Kd protein is the glycosylated derivative of the 61 Kd primary translation product. This is similar to the molecular weight predicted by the cDNA clone for BMP-6. In the absence of tunicamycin, the predominant protein secreted from COS-1 cells is the 65 Kd glycosylated, unprocessed clipped form of BMP-6. There are also peptides of 46 Kd and 20 Kd present at lower abundance than the 65 Kd that likely represent the processed pro and mature peptides, respectively.

### C. BMP-7 COS Expression

Intracellular BMP-7 protein in tunicamycin-treated COS-1 cells is detected as a doublet of 44 Kd and 46 Kd. In the absence of tunicamycin, proteins of 46 Kd and perhaps 48 Kd are synthesized. These likely represent glycosylated derivatives of the BMP-7 primary translation products. The 48 Kd protein is the major BMP species secreted from COS-1 cells, again suggesting inefficient cleavage of BMP-7 at the propeptide dibasic cleavage site.

### Example VIII

### CHO Cell Expression

DHFR deficient CHO cells (DUKX Bll) are transfected by electroporation with one of the BMP-5, BMP-6 or BMP-7 expression vectors described above, and selected for expression of DHFR by growth in nucleoside-free media. Other methods of transfection, including but not limited to CaPO₄ precipitation, protoplast fusion, microinjection, and lipofection, may also be employed. In order to obtain higher levels of expression more expediently, cells may be selected in nucleoside-free media supplemented with 5 nM, 20 nM or 100 nM MTX. Since the DHFR selectable marker is physically linked to the BMP cDNA as the second gene of a bicistronic coding region, cells which express DHFR should also express the BMP encoded within the upstream cistron. Either single clones, or pools of combined clones, are expanded and analyzed for expression of BMP protein. Cells are selected in stepwise increasing concentrations of MTX (5 nM, 20 nM, 100 nM, 500 nM, 2 uM, 10 uM, and 100 uM) in order to obtain cell lines which contain multiple copies of the expression vector DNA by virtue of gene amplification, and hence secrete large amounts of BMP protein.

Using standard techniques cell lines are screened for expression of BMP RNA, protein or activity, and high expressing cell lines are cloned or recloned at the appropriate level of selection to obtain a more homogeneous population of cells. The resultant cell line is then further characterized for BMP DNA sequences, and expression of BMP RNA and protein. Suitable cell lines can then be used for producing recombinant BMP protein.

### A. CHO Expression of BMP-5

The BMP-5 vector BMP5mix/pMT21#2 described above is transfected into CHO cells by electroporation, and cells are selected for expression of DHFR. Clonal cell lines are obtained from individual colonies selected stepwise for resistence to MTX, and analyzed for secretion of BMP-5 proteins. In some cases cell lines may be maintained as pools and cloned at later stages of MTX selection.

As described in Example V.B. the cDNA for BMP-5 encodes for a protein of approximately 52 Kd. Following processing within the cell that includes, but may not be limited to, propeptide cleavage, glycosylation, and dimer or multimer formation, multiple BMP-5 peptides are produced. There are at least 4 candidate peptides for processed forms of the BMP-5 protein discernable following SDS PAGE under reducing conditions; a 65 Kd peptide, a 35 Kd peptide, and a doublet of approximately 22 Kd molecular weight. Other less abundant BMP-5 peptides may also be present. By comparison to the processing of other related BMP molecules and the related protein TGF-beta, the 65 Kd protein likely represents unprocessed BMP-5, the 35 Kd species represents the propeptide, and the 22 Kd doublet represents the mature peptide.

Material from a BMP-5 cell line is analyzed in a 2-dimensional gel system. In the first dimension, proteins are electrophoresed under nonreducing conditions. The material is then reduced, and electrophoresed in a second polyacrylamide gel. Proteins that form disulfide-bonded dimers or multimers will run below a diagonal across the second reduced gel. Results from analysis of BMP-5 protein indicates that a significant amount of the mature BMP-5 peptides can form homodimers of approximately 30-35 Kd that reduce to the 22 Kd doublet observed in one dimensional reduced gels. A fraction of the mature peptides are apparently in a disulfide-bonded complex with the pro peptide. The amount of this complex is minor relative to the mature homodimer. In addition, some of the unprocessed protein can apparantly form homodimers or homomultimers.

### B. CHO Expression of BMP-6

The BMP-6 expression vector BMP6/pMT21 described above is transfered into CHO cells and selected for stable transformants via DHFR expression in a manner as described above in part A with relation to BMP-5. The mature active species of BMP-6 is contemplated to comprise amino acid #382 - #513 of Table IV. It is contemplated that secreted BMP-6 protein will be processed in a manner similar to that described above for BMP-5, other related BMP molecules and analogous to the processing of the related protein TGF-β [Gentry, et al.; Dernyck, et al., Supra.].

### C. CHO Expression of BMP-7

The BMP-7 expression vector BMP7/pMT21 described above is transfected into CHO cells and selected for stable transformants via DHFR expression in a manner as described above in relation to BMP-5. The mature active species of BMP-7 is contemplated to comprise amino acid #300-#431 of Table V. It is contemplated that secreted BMP-7 protein will processed in a manner similar to that described above for BMP-5, other related BMP molecules and analogous to the processing of the related protein TGF-β [Gentry, et al.; Dernyck, et al., Supra.].

### EXAMPLE IX

### Biological Activity of Expressed BMP Proteins

To measure the biological activity of the expressed BMP-5, BMP-6 and BMP-7 proteins obtained in Example VII and VIII above, the BMP proteins are recovered from the culture media and purified by isolating the BMP proteins from other proteinaceous materials with which they are co-produced, as well as from other contaminants. The proteins may be partially purified on a Heparin Sepharose column and further purified using standard purification techniques known to those skilled in the art.

For instance, post transfection conditioned medium supernatant collected from the cultures is concentrated approximately 10 fold by ultrafiltration on a YM 10 membrane and then dialyzed against 20mM Tris, 0.15 M NaCl, pH 7.4 (starting buffer). This material is then applied to a Heparin Sepharose column in starting buffer. Unbound proteins are removed by a wash of starting buffer, and bound proteins, including proteins of the invention, are desorbed by a wash of 20 mM Tris, 2.0 M NaCl, pH 7.4. The proteins bound by the Heparin column are concentrated approximately 10-fold on, for example, a Centricon 10 and the salt reduced by diafiltration with, for example, 0.1% trifluoroacetic acid. The appropriate amount of the resultant solution is mixed with 20 mg of rat matrix and then assayed for in vivo bone and/or cartilage formation activity by the Rosen-modified Sampath - Reddi assay. A mock transfection supernatant fractionation is used as a control.

Further purification may be achieved by preparative NaDodSO₄/PAGE [:aemmli, Nature 227:680-685 (1970)], for instance, approximately 300 µg of protein is applied to a 1.5-mm-thick 12.5% gel: recovery is be estimated by adding L-[³⁵S]methionine-labeled BMP protein purified over heparin-Sepharose as described above. Protein may be visualized by copper staining of an adjacent lane [Lee, et al., Anal. Biochem. 166:308-312 (1987)]. Appropriate bands are excised and extracted in 0.1% NaDodSO₄/20 mM Tris, pH 8.0. The supernatant may be acidified with 10% CF₃COOH to pH 3 and the proteins are desalted on 5.0 x 0.46 cm Vydac C₄ column (The Separations Group, Hesperia, CA) developed with a gradient of 0.1% CF₃COOH to 90% acetonitrile/0.1% CF₃COOH.

The implants containing rat matrix to which specific amounts of human BMP-5, BMP-6 or BMP-7 proteins of the invention have been added are removed from rats after approximately seven days and processed for histological evaluation. Representative sections from each implant are stained for the presence of new bone mineral with von Kossa and acid fuschin, and for the presence of cartilage-specific matrix formation using toluidine blue. The types of cells present within the section, as well as the extent to which these cells display phenotype are evaluated and scored as described in Example III.

Levels of activity may also be tested for host cell extracts. Purification is accomplished in a similar manner as described above except that 6 M urea is included in all the buffers.

The foregoing descriptions detail presently preferred embodiments of the present invention. Numerous modifications and variations in practice thereof are expected to occur to those skilled in the art upon consideration of these descriptions. Those modifications and variations are believed to be encompassed within the claims appended hereto.

## Claims (Claims for the following Contracting State(s): AT, BE, CH, DE, DK, FR, GB, IT, LI, LU, NL, SE)

1. A DNA sequence encoding a BMP protein having the ability to induce the formation of cartilage and/or bone, said DNA sequence being selected from the group consisting of:
(a) a DNA sequence encoding BMP-5,
(i) comprising the nucleotide positions #699 to #2060 of Table III;
(ii) comprising the nucleotide positions #1665 to #2060 of Table III;
(iii) comprising sequences which hybridise to the sequence of (i) under stringent hybridisation conditions and encode a protein having the biological properties of BMP-5;
(iv) comprising sequences which are degenerate or allelic with respect to a sequence of any one of sections (i) to (iii); or
(v) having the amino acid sequence with the amino acids #323 to #454 of Table III;
(b) a DNA sequence encoding BMP-6,
(i) comprising nucleotide positions #160 to #1698 of Table IV;
(ii) comprising nucleotide positions #1303 to #1698 of Table IV,
(iii) comprising sequences which hybridise to the sequence of (i) under stringent hybridisation conditions and encode a protein having the biological properties of BMP-6;
(iv) comprising sequences which are degenerate or allelic with respect to a sequence of any one of sections (i) to (iii); or
(v) having the amino acid sequence with the amino acids #382 to #513 of Table IV; and
(c) a DNA sequence encoding BMP-7,
(i) comprising nucleotide positions #994 to #1389 of Table V;
(ii) comprising sequences which hybridise to the sequence of (i) under stringent hybridisation conditions, encode an amino acid sequence with the N-terminal position corresponding to the amino acid position encoded by the codon starting with nucleotide position #994 and encode a protein having the biological properties of BMP-7;
(iii) comprising sequences which are degenerate or allelic with respect to a sequence of any one of sections (i) to (iii); or
(iv) having the amino acid sequence with the amino acids #300 to #431 of Table V.

2. A vector comprising a DNA sequence of claim 1 in operative association with an expression control sequence therefor.

3. A host cell transformed with the vector of claim 2.

4. The host cell according to claim 3, which is a mammalian cell.

5. The host cell according to claim 4, which is a CHO cell.

6. A protein exhibiting properties of BMP-5, BMP-6 or BMP-7, which is encoded by a DNA sequence of claim 1.

7. A protein exhibiting properties of BMP-5, BMP-6 or BMP-7, which is obtainable by the steps of:
(a) culturing in a suitable culture medium a host cell of any one of claims 3 to 5, and
(b) recovering, isolating and purifying said protein from said culture medium.

8. The protein of claim 6 or 7, further characterised by the ability to demonstrate the induction of cartilage and/or bone formation.

9. A method for producing a BMP protein, said method comprising the steps of:
(a) culturing in a suitable culture medium the transformed host cell of claims 4 to 6; and
(b) recovering, isolating and purifying said protein from said culture.

10. A protein exhibiting properties of BMP-7, which is obtainable by the steps of:
(a) culturing in a suitable culture medium a CHO cell transformed with a DNA sequence encoding amino acids #1 to #431 of Table V or with a DNA sequence comprising nucleotides #97 to #1389 of Table V; and
(b) recovering, isolating and purifying said BMP-7 protein from said culture medium.

11. A method for producing a BMP-7 protein, said method comprising the steps of:
(a) culturing in a suitable culture medium a CHO cell transformed with a DNA sequence encoding amino acids #1 to #431 of Table V or with a DNA sequence comprising nucleotides #97 to #1389 of Table V; and
(b) recovering, isolating and purifying said BMP-7 protein from said culture.

12. A pharmaceutical composition comprising a protein of any one of claims 6 to 8 or 10 in admixture with a pharmaceutically acceptable vehicle.

13. The composition of claim 12, further comprising a pharmaceutically acceptable matrix.

14. The composition of claim 13, wherein said matrix comprises hydroxyapatite, collagen, polylactic acid or tricalcium phosphate.

15. Use of a protein as defined in any one of claims 6 to 8 or 10 for the preparation of a pharmaceutical composition for the treatment of a patient in need of cartilage and/or bone formation.

16. Use of a protein as defined in any one of claims 6 to 8 or 10 for the preparation of a pharmaceutical composition for wound healing and tissue repair.

17. A DNA sequence comprising nucleotide #1 through #2153 as shown in Table III.

18. A DNA sequence comprising nucleotide #1 through #2923 as shown in Table IV.

19. A DNA sequence comprising nucleotide #1 through 1448 as shown in Table V.

20. A DNA sequence comprising the BMP-7 DNA sequence of ATCC deposit 68020.

## Claims (Claims for the following Contracting State(s): ES)

1. A method for preparing a DNA sequence encoding a BMP protein having the ability to induce the formation of cartilage and/or bone, said DNA sequence being selected from the group consisting of:
(a) a DNA sequence encoding BMP-5,
(i) comprising the nucleotide positions #699 to #2060 of Table III;
(ii) comprising the nucleotide positions #1665 to #2060 of Table III;
(iii) comprising sequences which hybridise to the sequence of (i) under stringent hybridisation conditions and encode a protein having the biological properties of BMP-5;
(iv) comprising sequences which are degenerate or allelic with respect to a sequence of any one of sections (i) to (iii); or
(v) having the amino acid sequence with the amino acids #323 to #454 of Table III;
(b) a DNA sequence encoding BMP-6,
(i) comprising nucleotide positions #160 to #1698 of Table IV;
(ii) comprising nucleotide positions #1303 to #1698 of Table IV,
(iii) comprising sequences which hybridise to the sequence of (i) under stringent hybridisation conditions and encode a protein having the biological properties of BMP-6;
(iv) comprising sequences which are degenerate or allelic with respect to a sequence of any one of sections (i) to (iii); or
(v) having the amino acid sequence with the amino acids #382 to #513 of Table IV; and
(c) a DNA sequence encoding BMP-7,
(i) comprising nucleotide positions #994 to #1389 of Table V;
(ii) comprising sequences which hybridise to the sequence of (i) under stringent hybridisation conditions, encode an amino acid sequence with the N-terminal position corresponding to the amino acid position encoded by the codon starting with nucleotide position #994 and encode a protein having the biological properties of BMP-7;
(iii) comprising sequences which are degenerate or allelic with respect to a sequence of any one of sections (i) to (iii); or
(iv) having the amino acid sequence with the amino acids #300 to #431 of Table V
said method comprising the following steps:
(a) construction of a cDNA library containing the target cDNA;
(b) screening of the cDNA library with a suitable probe, which is a fragment of the DNA encoding a bovine
BMP-5,BMP-6 or BMP-7 protein; and (c) isolating clones containing DNA encoding a BMP-5, BMP-6 or BMP-7 protein.

2. A method for the preparation of an expression vector comprising the insertion of a DNA sequence of claim 1 in operative association with an expression control sequence.

3. A host cell transformed with the vector of claim 2.

4. The host cell according to claim 3, which is a mammalian cell.

5. The host cell according to claim 4, which is a CHO cell.

6. A method for producing a BMP protein, said method comprising the steps of:
(a) culturing in a suitable culture medium the transformed host cell of claims 3 to 5; and
(b) recovering, isolating and purifying said protein from said culture.

7. The method of claim 6, wherein said protein is further characterised by the ability to demonstrate the induction of cartilage and/or bone formation.

8. A method for producing a BMP-7 protein, said method comprising the steps of:
(a) culturing in a suitable culture medium a CHO cell transformed with a DNA sequence encoding amino acids #1 to #431 of Table V or with a DNA sequence comprising nucleotides #97 to #1389 of Table V; and
(b) recovering, isolating and purifying said BMP-7 protein from said culture.

9. A method for the preparation of a pharmaceutical composition comprising combining a protein produced according to the method of any one of claims 6 to 8 with a pharmaceutically acceptable vehicle.

10. The method of claim 9, wherein said composition further comprises a pharmaceutically acceptable matrix.

11. The method of claim 10, wherein said matrix comprises hydroxyapatite, collagen, polylactic acid or tricalcium phosphate.

12. The method of any one of claims 9 to 11 wherein said pharmaceutical composition is for the treatment of a patient in need of cartilage and/or bone formation.

13. The method of any of claims 9 to 11 wherein said pharmaceutical composition is for wound healing and tissue repair.

14. A method for the preparation of a DNA sequence comprising nucleotide #1 through #2153 as shown in Table III, said method comprising the following steps:
(a) construction of a cDNA library containing the target cDNA;
(b) screening of the cDNA library with a suitable probe, which is a fragment of the DNA encoding a bovine
BMP-5 protein; and (c) isolating clones containing DNA encoding a BMP-5 protein.

15. A method for the preparation of a DNA sequence comprising nucleotide #1 through #2923 as shown in Table IV, said method comprising the following steps:
(a) construction of a cDNA library containing the target cDNA;
(b) screening of the cDNA library with a suitable probe, which is a fragment of the DNA encoding a bovine
BMP-6 protein; and (c) isolating clones containing DNA encoding a BMP-6 protein.

16. A method for the preparation of a DNA sequence comprising nucleotide #1 through 1448 as shown in Table V, said method comprising the following steps:
(a) construction of a cDNA library containing the target cDNA;
(b) screening of the cDNA library with a suitable probe, which is a fragment of the DNA encoding a bovine
BMP-7 protein; and (c) isolating clones containing DNA encoding a BMP-7 protein.

17. The method of claim 16, wherein the BMP-7 DNA sequence is contained in the clone ATCC deposit 68020.

## Patentansprüche (Patentansprüche für folgende(n) Vertragsstaat(en): AT, BE, CH, DE, DK, FR, GB, IT, LI, LU, NL, SE)

1. DNA-Sequenz, die ein BMP-Protein codiert, das die Fähigkeit besitzt, Knorpelund/oder Knochenbildung zu induzieren, wobei die DNA-Sequenz
(a) eine DNA-Sequenz, die BMP-5 codiert, und die
(i) die Nucleotidpositionen #699 bis #2060 in Tabelle III umfaßt;
(ii) die Nucleotidpositionen #1665 bis #2060 in Tabelle III umfaßt;
(iii) Sequenzen umfaßt, die mit der Sequenz nach (i) unter stringenten Hybridisierungsbedingungen hybridisieren und ein Protein codieren, das die biologischen Eigenschaften von BMP-5 besitzt;
(iv) degenerierte Sequenzen oder Allele einer Sequenz nach (i) bis (iii) umfaßt; oder
(v) BMP-5 codiert, das die Aminosäuresequenz von Position #323 bis #454 in Tabelle III besitzt;
(b) eine DNA-Sequenz, die BMP-6 codiert, und die
(i) die Nucleotidpositionen #160 bis #1698 in Tabelle IV umfaßt;
(ii) die Nucleotidpositionen #1303 bis #1698 in Tabelle IV umfaßt;
(iii) Sequenzen umfaßt, die mit der Sequenz nach (i) unter stringenten Hybridisierungsbedingungen hybridisieren und ein Protein codieren, das die biologischen Eigenschaften von BMP-6 besitzt;
(iv) degenerierte Sequenzen oder Allele einer Sequenz nach (i) bis (iii) umfaßt; oder
(v) BMP-6 codiert, das die Aminosäuresequenz von Position #382 bis #513 in Tabelle IV besitzt; oder
(c) eine DNA-Sequenz, die BMP-7 codiert, und die
(i) die Nucleotidpositionen #994 bis #1389 in Tabelle V umfaßt;
(ii) Sequenzen umfaßt, die mit der Sequenz nach (i) unter stringenten Hybridisierungsbedingungen hybridisieren, die eine Aminosäuresequenz codieren, deren N-Terminus der Aminosäure entspricht, die das Codon mit der Nucleotidposition #994 beginnend codiert, und die ein Protein codieren, das die biologischen Eigenschaften von BMP-7 besitzt;
(iii) degenerierte Sequenzen oder Allele einer Sequenz nach (i) bis (iii) umfaßt; oder
(iv) BMP-7 codiert, das die Aminosäuresequenz von Position #300 bis #431 in Tabelle V besitzt,
ist.

2. Vektor, der eine DNA-Sequenz nach Anspruch 1 in funktioneller Verknüpfung mit einer Expressionskontrollsequenz umfaßt.

3. Wirtszelle, die mit dem Vektor nach Anspruch 2 transformiert ist.

4. Wirtszelle nach Anspruch 3, die eine Säugerzelle ist.

5. Wirtszelle nach Anspruch 4, die eine CHO-Zelle ist.

6. Protein, das Eigenschaften von BMP-5, BMP-6 oder BMP-7 aufweist und das von einer DNA-Sequenz nach Anspruch 1 codiert wird.

7. Protein, das Eigenschaften von BMP-5, BMP-6 oder BMP-7 aufweist und durch folgende Schritte erhältlich ist:
(a) Züchtung einer Wirtszelle nach einem der Ansprüche 3 bis 5 in einem geeigneten Kulturmedium, und
(b) Gewinnung, Isolierung und Reinigung des Proteins aus dem Kulturmedium.

8. Protein nach Anspruch 6 oder 7, weiter gekennzeichnet durch die Fähigkeit, die Induktion von Knorpel- und/oder Knochenbildung zu bewirken.

9. Verfahren zur Herstellung eines BMP-Proteins, das folgende Schritte umfaßt:
(a) Züchtung der transformierten Wirtszelle nach Anspruch 4 bis 6 in einem geeigneten Kulturmedium, und
(b) Gewinnung, Isolierung und Reinigung des Proteins aus dem Kulturmedium.

10. Protein, das Eigenschaften von BMP-7 aufweist und durch folgende Schritte erhältlich ist:
(a) Züchtung einer CHO-Zelle in einem geeigneten Kulturmedium, die mit einer DNA-Sequenz transformiert ist, die die Aminosäuren #1 bis #431 in Tabelle V codiert, oder mit einer DNA-Sequenz, die die Nucleotide #97 bis #1389 in Tabelle V umfaßt, und
(b) Gewinnung, Isolierung und Reinigung des BMP-7-Proteins aus dem Kulturmedium.

11. Verfahren zur Herstellung eines BMP-7-Proteins, das folgende Schritte umfaßt:
(a) Züchtung einer CHO-Zelle in einem geeigneten Kulturmedium, die mit einer DNA-Sequenz transformiert ist, die die Aminosäuren #1 bis #431 in Tabelle V codiert, oder mit einer DNA-Squenz, die die Nucleotide #97 bis #1389 in Tabelle V umfaßt, und
(b) Gewinnung, Isolierung und Reinigung des BMP-7-Proteins aus dem Kulturmedium.

12. Arzneimittel, das ein Protein nach einem der Ansprüche 6 bis 8 oder 10 in einem Gemisch mit einem pharmazeutisch verträglichen Träger umfaßt.

13. Arzneimittel nach Anspruch 12, das weiter eine pharmazeutisch verträgliche Matrix umfaßt.

14. Arzneimittel nach Anspruch 13, wobei die Matrix Hydroxyapatit, Kollagen, Polymilchsäure oder Tricalciumphosphat umfaßt.

15. Verwendung eines Proteins gemäß der Definition in einem der Ansprüche 6 bis 8 oder 10 für die Herstellung eines Arzneimittels zur Behandlung eines Patienten, der Knorpel- und/oder Knochenbildung benötigt.

16. Verwendung eines Proteins gemäß der Definition in einem der Ansprüche 6 bis 8 oder 10 für die Herstellung eines Arzneimittels zur Wundheilung oder Wiederherstellung von Gewebe.

17. DNA-Sequenz, die die Nucleotide #1 bis #2153 wie in Tabelle III gezeigt umfaßt.

18. DNA-Sequenz, die die Nucleotide #1 bis #2923 wie in Tabelle IV gezeigt umfaßt.

19. DNA-Sequenz, die die Nucleotide #1 bis #1448 wie in Tabelle V gezeigt umfaßt.

20. DNA-Sequenz, die die BMP-7-DNA-Sequenz der ATCC-Hinterlegungsnummer 68020 umfaßt.

## Patentansprüche (Patentansprüche für folgende(n) Vertragsstaat(en): ES)

1. Verfahren zur Herstellung einer DNA-Sequenz, die ein BMP-Protein codiert, das die Fähigkeit besitzt, Knorpel- und/oder Knochenbildung zu induzieren, wobei die DNA-Sequenz
(a) eine DNA-Sequenz, die BMP-5 codiert, und die
(i) die Nucleotidpositionen #699 bis #2060 in Tabelle III umfaßt;
(ii) die Nucleotidpositionen #1665 bis #2060 in Tabelle III umfaßt;
(iii) Sequenzen umfaßt, die mit der Sequenz nach (i) unter stringenten Hybridisierungsbedingungen hybridisieren und ein Protein codieren, das die biologischen Eigenschaften von BMP-5 besitzt;
(iv) degenerierte Sequenzen oder Allele einer Sequenz nach (i) bis (iii) umfaßt; oder
(v) BMP-5 codiert, das die Aminosäuresequenz von Position #323 bis #454 in Tabelle III besitzt;
(b) eine DNA-Sequenz, die BMP-6 codiert, und die
(i) die Nucleotidpositionen #160 bis #1698 in Tabelle IV umfaßt;
(ii) die Nucleotidpositionen #1303 bis #1698 in Tabelle IV umfaßt;
(iii) Sequenzen umfaßt, die mit der Sequenz nach (i) unter stringenten Hybridisierungsbedingungen hybridisieren und ein Protein codieren, das die biologischen Eigenschaften von BMP-6 besitzt;
(iv) degenerierte Sequenzen oder Allele einer Sequenz nach (i) bis (iii) umfaßt; oder
(v) BMP-6 codiert, das die Aminosäuresequenz von Position #382 bis #513 in Tabelle IV besitzt; oder
(c) eine DNA-Sequenz, die BMP-7 codiert, und die
(i) die Nucleotidpositionen #994 bis #1389 in Tabelle V umfaßt;
(ii) Sequenzen umfaßt, die mit der Sequenz nach (i) unter stringenten Hybridisierungsbedingungen hybridisieren, die eine Aminosäuresequenz codieren, deren N-Terminus der Aminosäure entspricht, die das Codon mit der Nucleotidposition #994 beginnend codiert, und die ein Protein codieren, das die biologischen Eigenschaften von BMP-7 besitzt;
(iii) degenerierte Sequenzen oder Allele einer Sequenz nach (i) bis (iii) umfaßt; oder
(iv) BMP-7 codiert, das die Aminosäuresequenz von Position #300 bis #431 in Tabelle V besitzt,
ist und das Verfahren folgende Schritte umfaßt:
(a) Konstruktion einer cDNA-Bank, die die gesuchte cDNA enthält;
(b) Absuchen der cDNA-Bank mit einer geeigneten Sonde, die ein Fragment der DNA ist, die ein BMP-5-, BMP-6- oder BMP-7-Protein vom Rind codiert; und
(c) Isolierung von Clonen, die DNA enthalten, die ein BMP-5-, BMP-6- oder BMP-7-Protein codiert.

2. Verfahren zur Herstellung eines Expressionsvektors, umfassend die Insertion einer DNA-Sequenz nach Anspruch 1 in funktioneller Verknüpfung mit einer Expressionskontrollsequenz.

3. Wirtszelle, die mit dem Vektor nach Anspruch 2 transformiert ist.

4. Wirtszelle nach Anspruch 3, die eine Säugerzelle ist.

5. Wirtszelle nach Anspruch 4, die eine CHO-Zelle ist.

6. Verfahren zur Herstellung eines BMP-Proteins, wobei das Verfahren folgende Schritte umfaßt:
(a) Züchtung der transformierten Wirtszelle nach einem der Ansprüche 3 bis 5 in einem geeigneten Kulturmedium, und
(b) Gewinnung, Isolierung und Reinigung des Proteins aus dem Kulturmedium.

7. Verfahren nach Anspruch 6, wobei das Protein weiter gekennzeichnet ist durch die Fähigkeit, die Induktion von Knorpel- und/oder Knochenbildung zu bewirken.

8. Verfahren zur Herstellung eines BMP-7-Proteins, das folgende Schritte umfaßt:
(a) Züchtung einer CHO-Zelle in einem geeigneten Kulturmedium, die mit einer DNA-Sequenz transformiert ist, die die Aminosäuren #1 bis #431 in Tabelle V codiert, oder mit einer DNA-Sequenz, die die Nucleotide #97 bis #1389 in Tabelle V umfaßt, und
(b) Gewinnung, Isolierung und Reinigung des BMP-7-Proteins aus dem Kulturmedium.

9. Verfahren zur Herstellung eines Arzneimittels, das das Mischen eines Proteins, hergestellt gemäß dem Verfahren nach einem der Ansprüche 6 bis 8, mit einem pharmazeutisch verträglichen Träger umfaßt.

10. Verfahren nach Anspruch 9, wobei das Arzneimittel weiter eine pharmazeutisch verträgliche Matrix umfaßt.

11. Verfahren nach Anspruch 10, wobei die Matrix Hydroxyapatit, Kollagen, Polymilchsäure oder Tricalciumphosphat umfaßt.

12. Verfahren nach einem der Ansprüche 9 bis 11, wobei das Arzneimittel zur Behandlung eines Patienten ist, der Knorpel- und/oder Knochenbildung benötigt.

13. Verfahren nach einem der Ansprüche 9 bis 11, wobei das Arzneimittel zur Wundheilung oder Wiederherstellung von Gewebe ist.

14. Verfahren zur Herstellung einer DNA-Sequenz, die die Nucleotide #1 bis #2153 wie in Tabelle III gezeigt umfaßt, wobei das Verfahren die folgenden Schritte umfaßt:
(a) Konstruktion einer cDNA-Bank, die die gesuchte cDNA enthält;
(b) Absuchen der cDNA-Bank mit einer geeigneten Sonde, die ein Fragment der DNA ist, die ein BMP-5-Protein vom Rind codiert; und
(c) Isolierung von Clonen, die DNA enthalten, die ein BMP-5-Protein codiert.

15. Verfahren zur Herstellung einer DNA-Sequenz, die die Nucleotide #1 bis #2923 wie in Tabelle IV gezeigt umfaßt, wobei das Verfahren die folgenden Schritte umfaßt:
(a) Konstruktion einer cDNA-Bank, die die gesuchte cDNA enthält;
(b) Absuchen der cDNA-Bank mit einer geeigneten Sonde, die ein Fragment der DNA ist, die ein BMP-6-Protein vom Rind codiert; und
(c) Isolierung von Clonen, die DNA enthalten, die ein BMP-6-Protein codiert.

16. Verfahren zur Herstellung einer DNA-Sequenz, die die Nucleotide #1 bis #1448 wie in Tabelle V gezeigt umfaßt, wobei das Verfahren die folgenden Schritte umfaßt:
(a) Konstruktion einer cDNA-Bank, die die gesuchte cDNA enthält;
(b) Absuchen der cDNA-Bank mit einer geeigneten Sonde, die ein Fragment der DNA ist, die ein BMP-7-Protein vom Rind codiert; und
(c) Isolierung von Clonen, die DNA enthalten, die ein BMP-7-Protein codiert.

17. Verfahren nach Anspruch 16, wobei die BMP-7-DNA-Sequenz in dem Clon mit der ATCC-Hinterlegungsnummer 68020 enthalten ist.

## Revendications (Revendications pour l'(les) Etat(s) contractant(s) suivant(s): AT, BE, CH, DE, DK, FR, GB, IT, LI, LU, NL, SE)

1. Séquence d'ADN codant une protéine BMP ayant la capacité d'induire la formation de cartilage et/ou d'os, cette séquence d'ADN étant choisie parmi le groupe comprenant :
(a) une séquence d'ADN codant BMP-5,
(i) comprenant les positions nucléotidiques #699 à #2060 du tableau III,
(ii) comprenant les positions nucléotidiques #1665 à #2060 du tableau III,
(iii) comprenant des séquences qui s'hybrident à la séquence de (i) dans des conditions d'hybridation rigoureuses et qui codent une protéine ayant les propriétés biologiques de BMP-5,
(iv) comprenant des séquences qui sont dégénérées ou allèles par rapport à une séquence de l'une quelconque des sections (i) à (iii), ou
(v) comportant la séquence d'acides aminés avec les acides aminés #323 à #454 du tableau III,
(b) une séquence d'ADN codant BMP-6,
(i) comprenant des positions nucléotidiques #160 à #1698 du tableau IV,
(ii) comprenant des positions nucléotidiques #1303 à #1698 du tableau IV,
(iii) comprenant des séquences qui s'hybrident à la séquence de (i) dans des conditions d'hybridation rigoureuses et qui codent une protéine ayant les propriétés biologiques de BMP-6,
(iv) comprenant des séquences qui sont dégénérées ou allèles par rapport à une séquence de l'une quelconque des sections (i) à (iii), ou
(v) comportant la séquence d'acides aminés avec les acides aminés #382 à #513 du tableau IV, et
(c) une séquence d'ADN codant BMP-7,
(i) comprenant des positions nucléotidiques #994 à #1389 du tableau V,
(ii) comprenant des séquences qui s'hybrident à la séquence de (i) dans des conditions d'hybridation rigoureuses, codent une séquence d'acides aminés avec la position N-terminale correspondant à la position d'acide aminé codée par le codon initiant avec la position nucléotidique #994 et codent une protéine ayant les propriétés biologiques de BMP-7,
(iii) comprenant des séquences qui sont dégénérées ou allèles par rapport à une séquence de l'une quelconque des sections (i) à (iii), ou
(iv) comportant la séquence d'acides aminés avec les acides aminés #300 à #431 du tableau V.

2. Vecteur comprenant une séquence d'ADN de la revendication 1 en association fonctionnelle avec une séquence de contrôle d'expression de celle-ci.

3. Cellule hôte transformée par le vecteur de la revendication 2.

4. Cellule hôte suivant la revendication 3, qui est une cellule de mammifère.

5. Cellule hôte suivant la revendication 4 qui est une cellule CHO.

6. Protéine montrant des propriétés de BMP-5, BMP-6 ou BMP-7, qui est codée par une séquence d'ADN suivant la revendication 1.

7. Protéine montrant des propriétés de BMP-5, BMP-6 ou BMP-7, qui peut être obtenue par les étapes :
(a) de culture dans un milieu de culture approprié d'une cellule hôte suivant l'une quelconque des revendications 3 à 5, et
(b) de récupération, d'isolement et de purification de cette protéine à partir de ce milieu de culture.

8. Protéine suivant l'une des revendications 6 et 7, caractérisée en outre par la capacité de démontrer l'induction de formation de cartilage et/ou d'os.

9. Procédé de production de protéine BMP, ce procédé comprenant les étapes :
(a) de culture dans yn milieu de culture approprié de la cellule hôte transformée suivant l'une des revendications 4 à 6, et
(b) de récupération, d'isolement et de purification de cette protéine à partir de cette culture.

10. Protéine montrant des propriétés de BMP-7, qui peut être obtenue par les étapes :
(a) de culture dans un milieu de culture approprié d'une cellule CHO transformée par une séquence d'ADN codant des acides aminés #1 à #431 du tableau V ou par une séquence d'ADN comprenant des nucléotides #97 à #1389 du tableau V, et
(b) de récupération, d'isolement et de purification de ladite protéine BMP-7 à partir de ce milieu de culture.

11. Procédé de production d'une protéine BMP-7, ce procédé comprenant les étapes :
(a) de culture dans un milieu de culture approprié d'une cellule CHO transformée par une séquence d'ADN codant des acides aminés #1 à #431 du tableau V ou par une séquence d'ADN comprenant des nucléotides #97 à #1389 du tableau V, et
(b) de récupération, d'isolement et de purification de cette protéine BMP-7 à partir de cette culture.

12. Composition pharmaceutique comprenant une protéine suivant l'une quelconque des revendications 6 à 8 ou 10 en mélange avec un support pharmaceutiquement acceptable.

13. Composition suivant la revendication 12, comprenant en outre une matrice pharmaceutiquement acceptable.

14. Composition suivant la revendication 13, caractérisée en ce que ladite matrice comprend de l'hydroxyapatite, du collagène, de l'acide polylactique ou du phosphate tricalcique.

15. Utilisation d'une protéine suivant l'une quelconque des revendications 6 à 8 ou 10 pour la préparation d'une composition pharmaceutique destinée au traitement d'un patient ayant besoin de formation de cartilage et/ou d'os.

16. Utilisation d'une protéine suivant l'une quelconque des revendications 6 à 8 ou 10 pour la préparation d'une composition pharmaceutique destinée à la cicatrisation d'une blessure et à la réparation d'un tissu.

17. Séquence d'ADN comprenant des nucléotides #1 à #2153 comme illustré dans le tableau III.

18. Séquence d'ADN comprenant des nucléotides #1 à #2923 comme illustré dans le tableau IV.

19. Séquence d'ADN comprenant des nucléotides #1 à #1448, comme illustré dans le tableau V.

20. Séquence d'ADN comprenant la séquence d'ADN de BMP-7 du dépôt ATCC 68020.

## Revendications (Revendications pour l'(les) Etat(s) contractant(s) suivant(s): ES)

1. Procédé de préparation d'une séquence d'ADN codant une protéine BMP ayant la capacité d'induire la formation de cartilage et/ou d'os, cette séquence d'ADN étant choisie parmi le groupe comprenant :
(a) une séquence d'ADN codant BMP-5,
(i) comprenant les positions nucléotidiques #699 à #2060 du tableau III,
(ii) comprenant les positions nucléotidiques #1665 à #2060 du tableau III,
(iii) comprenant des séquences qui s'hybrident à la séquence de (i) dans des conditions d'hybridation rigoureuses et qui codent une protéine ayant les propriétés biologiques de BMP-5,
(iv) comprenant des séquences qui sont dégénérées ou allèles par rapport à une séquence de l'une quelconque des sections (i) à (iii), ou
(v) comportant la séquence d'acides aminés avec les acides aminés #323 à #454 du tableau III,
(b) une séquence d'ADN codant BMP-6,
(i) comprenant des positions nucléotidiques #160 à #1698 du tableau IV,
(ii) comprenant des positions nucléotidiques #1303 à #1698 du tableau IV,
(iii) comprenant des séquences qui s'hybrident à la séquence de (i) dans des conditions d'hybridation rigoureuses et qui codent une protéine ayant les propriétés biologiques de BMP-6,
(iv) comprenant des séquences qui sont dégénérées ou allèles par rapport à une séquence de l'une quelconque des sections (i) à (iii), ou
(v) comportant la séquence d'acides aminés avec les acides aminés #382 à #513 du tableau IV, et
(c) une séquence d'ADN codant BMP-7,
(i) comprenant des positions nucléotidiques #994 à #1389 du tableau V,
(ii) comprenant des séquences qui s'hybrident à la séquence de (i) dans des conditions d'hybridation rigoureuses, codent une séquence d'acides aminés avec la position N-terminale correspondant à la position d'acide aminé codée par le codon initiant avec la position nucléotidique #994 et codent une protéine ayant les propriétés biologiques de BMP-7,
(iii) comprenant des séquences qui sont dégénérées ou allèles par rapport à une séquence de l'une quelconque des sections (i) à (iii), ou
(iv) comportant la séquence d'acides aminés avec les acides aminés #300 à #431 du tableau V,
ce procédé comprenant les étapes suivantes :
(a) de construction d'une bibliothèque d'ADNc contenant l'ADNc cible,
(b) de passage au crible de la bibliothèque d'ADNc par une sonde appropriée, qui est un fragment de l'ADN codant une protéine BMP-5, BMP-6 ou BMP-7 bovine, et
(c) d'isolement des clones contenant de l'ADN codant une protéine BMP-5, BMP-6 ou BMP-7.

2. Procédé de préparation d'un vecteur d'expression comprenant l'insertion d'une séquence d'ADN suivant la revendication 1 en association fonctionnelle avec une séquence de contrôle d'expression.

3. Cellule hôte transformée par le vecteur suivant la revendication 2.

4. Cellule hôte suivant la revendication 3, qui est une cellule de mammifère.

5. Cellule hôte suivant la revendication 4, qui est une cellule CHO.

6. Procédé de production de protéine BMP, ce procédé comprenant les étapes
(a) de culture dans un milieu de culture approprié de la cellule hôte transformée suivant l'une des revendications 3 à 5, et
(b) de récupération, d'isolement et de purification de cette protéine à partir de cette culture.

7. Procédé suivant la revendication 6, dans lequel la protéine est en outre caractérisée par la capacité de démontrer l'induction de formation de cartilage et/ou d'os.

8. Procédé de production d'une protéine BMP-7, ce procédé comprenant les étapes :
(a) de culture dans un milieu de culture approprié d'une cellule CHO transformée par une séquence d'ADN codant des acides aminés # 1 à # 431 du Tableau V ou par une séquence d'ADN comprenant des nucléotides # 97 à # 1389 du Tableau V, et
(b) de récupération, d'isolement et de purification de cette protéine BMP-7 à partir de cette culture.

9. Procédé de préparation d'une composition pharmaceutique comprenant une combinaison d'une protéine produite conformément au procédé suivant l'une quelconque des revendications 6 à 8 avec un support pharmaceutiquement acceptable.

10. Procédé suivant la revendication 9, caractérisé en ce que la composition comprend en outre une matrice pharmaceutiquement acceptable.

11. Procédé suivant la revendication 10, caractérisé en ce que la matrice comprend de l'hydroxyapatite, du collagène, de l'acide polylactique ou du phosphate tricalcique.

12. Procédé suivant l'une quelconque des revendications 9 à 11, caractérisé en ce que la composition pharmaceutique est destinée au traitement d'un patient ayant besoin de formation de cartilage et/ou d'os.

13. Procédé suivant l'une quelconque des revendications 9 à Il, caractérisé en ce que la composition pharmaceutique est destinée à la cicatrisation d'une blessure et à la réparation d'un tissu.

14. Procédé de préparation d'une séquence d'ADN comprenant des nucléotides #1 à #2153 comme illustré dans le Tableau III, ce procédé comprenant les étapes suivantes :
(a) de construction d'une bibliothèque d'ADNc contenant l'ADNc cible,
(b) de passage au crible de la bibliothèque d'ADNc par une sonde appropriée, qui est un fragment de l'ADNc codant une protéine BMP-5 bovine, et
(c) d'isolement de clones contenant de l'ADN codant une protéine BMP-5.

15. Procédé de préparation d'une séquence d'ADN comprenant des nucléotides #1 à #2923 comme illustré dans le Tableau IV, ce procédé comprenant les étapes suivantes :
(a) de construction d'une bibliothèque d'ADNc contenant l'ADNc cible,
(b) de passage au crible de la bibliothèque d'ADNc par une sonde appropriée, qui est un fragment de l'ADN codant une protéine BMP-6 bovine, et
(c) d'isolement de clones contenant de l'ADN codant une protéine BMP-6.

16. Procédé de préparation d'une séquence d'ADN comprenant des nucléotides #1 à #1448, comme illustré dans le Tableau V, ce procédé comprenant les étapes suivantes :
(a) de construction d'une bibliothèque d'ADNc contenant l'ADNc cible,
(b) de passage au crible de la bibliothèque d'ADNc par une sonde appropriée, qui est un fragment de l'ADN codant une protéine BMP-7 bovine, et
(c) d'isolement de clones contenant de l'ADN codant une protéine BMP-7.

17. Procédé suivant la revendication 16, caractérisé en ce que la séquence d'ADN de BMP-7 est contenue dans le dépôt ATCC 68020 de clone.
